# EUROPEAN PATENT SPECIFICATION

(11) **EP 1 255 852 B1**
(45) Date of publication and mention of the grant of the patent: **10.12.2008**
(21) Application number: 01908398.9
(22) Date of filing: 15.02.2001
(51) Int. Cl.: C12Q 1/04, G01N 33/53, C12N 15/00

(54) **IMMUNOASSAY AND DIAGNOSTIC REAGENT FOR MALARIA**
IMMUNOASSAY UND DIAGNOSTISCHES REAGENZ FÜR MALARIA
REACTIF DE DOSAGE IMMUNOLOGIQUE ET DE DIAGNOSTIC POUR LE PALUDISME

(30) Priority: 17.02.2000 KR 2000007648; 17.02.2000 KR 2000007649; 17.02.2000 KR 2000007650; 10.03.2000 KR 2000012172; 08.08.2000 KR 2000045806
(43) Date of publication of application: 13.11.2002
(73) Proprietor: LG Chem Investment, Ltd, Seoul 150-721 (KR)
(72) Inventor: LIM, Kook-Jin, Koyang-si, Kyungki-do 412-270 (KR); SHON, Mi-Jin, Daejeon-si 305-755 (KR); YOO, Seung-Bum, Daejeon-si 302-768 (KR); LEE, Sang-Ik, Daejeon-si 305-811 (KR); OH, Jae-Hoon, Daejeon-si 305-752 (KR); LEE, Seung-Won, Sungnam-si, Kyungki-do 463-830 (KR); KIM, Hyung-Cheol, Seoul 136-100 (KR)
(74) Representative: HOFFMANN EITLE
(86) International application number: PCT/KR2001/000229
(87) International publication number: WO 2001/061032

(56) References cited:
- WO-A-97/30158
- WO-A1-97/26911
- SOARES I S ET AL: "LONGEVITY OF NATURALLY ACQUIRED ANTIBODY RESPONSES TO THE N- AND C-TERMINAL REGIONS OF PLASMODIUM VIVAX MEROZOITE SURFACE PROTEIN 1" AMERICAN JOURNAL OF TROPICAL MEDICINE & HYGIENE, LAWRENCE, KS, US, vol. 60, no. 3, 1999, pages 357-363, XP009028883 ISSN: 0002-9637
- TYAGI P ET AL: "NATURALLY OCCURRING PLASMODIA-SPECIFIC CIRCULATING IMMUNE COMPLEXES IN INDIVIDUALS OF MALARIA ENDEMIC AREAS IN INDIA" INDIAN JOURNAL OF MALARIOLOGY, MALARIA RESEARCH CENTER, DEHLI, IN, vol. 36, no. 1/2, March 1999 (1999-03), pages 12-18, XP009028923
- RAY K ET AL: "EVALUATION OF INDIRECT IMMUNOFLUORESCENT ANTIBODY TEST FOR DETECTION OF IGM SPECIFIC ANTIBODIES IN MALARIA" JOURNAL OF COMMUNICABLE DISEASES, INDIA SOCIETY FOR MALARIA AND OTHER COMMUNICABLE DISEASES, DEL, IN, vol. 24, no. 2, June 1992 (1992-06), pages 82-86, XP009028938 ISSN: 0019-5138
- GIBSON H L ET AL: "STRUCTURE AND EXPRESSION OF THE GENE FOR PV200 A MAJOR BLOOD-STAGE SURFACE ANTIGEN OF PLASMODIUM-VIVAX" MOLECULAR AND BIOCHEMICAL PARASITOLOGY, vol. 50, no. 2, 1992, pages 325-334, XP000199300 ISSN: 0166-6851
- GENTZ R ET AL: "MAJOR SURFACE ANTIGEN P190 OF PLASMODIUM FALCIPARUM: DETECTION OF COMMON EPITOPES PRESENT IN A VARIETY OF PLASMODIA ISOLATES" EMBO JOURNAL, OXFORD UNIVERSITY PRESS, SURREY, GB, vol. 7, no. 1, 1988, pages 225-230, XP002057622 ISSN: 0261-4189
- SOARES ET AL.: 'Acquired immune responses to the N- and C-terminal regions of plasmodium vivax merozoite surface protein 1 in individuals exposed to malaria' INFECT. IMMUN. vol. 65, 1997, pages 1606 - 1614, XP002972558
- PAN ET AL: 'Vaccine candidate MSP-1 from plasmodium falciparum: a redesigned 4917 bp polynucleotide enables synthesis and isolation of full-length protein from escherichia coli and mammalian cells' NUCLEIC ACIDS RES. vol. 27, 15 February 1999, pages 1094 - 1103, XP000953100
- FRUH ET AL: 'A new tool for the serodiagnosis of acute plasmodium falciparum malaria in individuals with promary infection' J. IMMUNOL. METHODS vol. 122, 15 August 1989, pages 25 - 32, XP002972559
- SANCHEZ ET AL: 'Antibody response to plasmodium vivax antigens in human malaria' AM. J. TROP. MED. HYG. vol. 50, March 1994, pages 329 - 338, XP002973046
- SOARES ET AL: 'Antibody response to the N and C-terminal regions of the plasmodium vivax merozoite surface protein 1 in individuals living in an area of exclusive transmission of P. vivax malaria in the north of Brazil' ACTA TROP. vol. 72, 15 January 1999, pages 13 - 24, XP002972552
- MAIER ET AL: 'Serodiagnosis of malaria. Plasmodium berghei and P. falciparum as antigen for the indirect immune of fluorescence test' IMMUN. INFECT. vol. 7, June 1979, pages 75 - 82, XP002973070
- KASLOW ET AL: 'Expression and immunogenicity of the C-terminus of a major blood-stage surface protein of plasmodium vivax, Pv200(19), secreted from saccharomyces cerevisiae' IMMUNOL. LETT. vol. 51, July 1996, pages 187 - 189, XP002972560
- KASLOW ET AL: 'Expression and antigenicity of plasmodium falciparum major merozoite surface protein (MSP1(19)) variants secreted from saccharomyces cerevisiae' MOL. BIOCHEM. PARASITOL. vol. 63, 1994, pages 283 - 289, XP000603953
- DELCARDAYRE ET AL: 'Engineering ribonucleas A: production, purification and characterization of wild-type enzyme and mutants at G1n11' PROTEIN ENG. vol. 8, 1995, pages 261 - 273, XP002972561
- WITZGALL ET AL: 'A mammalian expression vector for the expression of GAL4 fusion proteins with an epitope tag and histidine tail' ANAL. BIOMCHEM. vol. 223, 1994, pages 291 - 298, XP002972562
- DATABASE GENBANK [Online] XP002972501 Retrieved from NCBI Database accession no. AAC37236
- HUW DAVIES: 'Introductory immunology', 1997, CHAPMAN & HALL pages 381 - 383, XP008041000
- PARK ET AL: 'Naturally acquired antibody responses to the C-terminal region of merozoite surface protein 1 of plasmodium vivax in Korea' CLIN. DIAGN. LAB. IMMUNOL. vol. 8, 2001, pages 14 - 20, XP002972563

### Sequence Listing

<110> Lg Chemical Ltd.
<120> IMMUNOASSAY AND DIAGNOSTIC REAGENT FOR MALARIA
<130> Pc007208LG
<150> KR 10-2000-7648
   <151> 2000-02-17
<150> KR 10-2000-7649
   <151> 2000-02-17
<150> KR 10-2000-7650
   <151> 2000-02-17
<150> KR 10-2000-12172
   <151> 2000-03-10
<150> KR 10-2000-45806
   <151> 2000-08-08
<160> 13
<170> KopatentIn 1.71
<210> 1
   <211> 108
   <212> PRT
   <213> Plasmodium vivax
<220>
   <221> PEPTIDE
   <222> (1) .. (108)
   <223> Carboxy-terminal polypeptide of Merozoit surface protein of Plasmodium vivax
<400> 1
   Asn Glu Ser Lys Glu Ile Leu Ser Gln Leu Leu Asn Val Gln Thr Gln
   1               5                   10                  15
   Leu Leu Thr Met Ser Ser Glu His Thr Cys Ile Asp Thr Asn Val Pro
   20 25 30
   Asp Asn Ala Ala Cys Tyr Arg Tyr Leu Asp Gly Thr Glu Glu Trp Arg
   35 40 45
   Cys Leu Leu Thr Phe Lys Glu Glu Gly Gly Lys Cys Val Pro Ala Ser
   50 55 60
   Asn Val Thr Cys Lys Asp Asn Asn Gly Gly Cys Ala Pro Glu Ala Glu
   65 70 75 80
   Cys Lys Met Thr Asp Ser Asn Lys Ile Val Cys Lys Cys Thr Lys Glu
   85 90 95
   Gly Ser Glu Pro Leu Phe Glu Gly Val Phe Cys Ser
   100 105
<210> 2
   <211> 31
   <212> DNA
   <213> Artificial Sequence
<220>
   <223> specific PCR primer for MSP protein
<400> 2
   aaaaaagtcg acgccaaaaa ggccgagctg g 31
<210> 3
   <211> 31
   <212> DNA
   <213> Artificial Sequence
<220>
   <223> specific PCR primer for MSP protein
<400> 3
   aaaaaagtcg acgagaagta cctcccgttc c 31
<210> 4
   <211> 31
   <212> DNA
   <213> Artificial Sequence
<220>
   <223> specific PCR primer for MSP protein
<400> 4
   aaaaaaatcg atttaaagct ccatgcacag g 31
<210> 5
   <211> 30
   <212> DNA
   <213> Artificial Sequence
<220>
   <223> specific PCR primer for MSP protein
<400> 5
   tctctagata agagaaacga gtccaaggaa 30
<210> 6
   <211> 45
   <212> DNA
   <213> Artificial Sequence
<220>
   <223> specific PCR primer for MSP protein
<400> 6
   aaactcgagt cagtggtggt ggtggtggtg gctacagaaa actcc 45
<210> 7
   <211> 24
   <212> DNA
   <213> Artificial Sequence
<220>
   <223> specific PCR primer for MSP protein
<400> 7
   aaaaagctta tgagatttcc ttca 24
<210> 8
   <211> 20
   <212> DNA
   <213> Artificial Sequence
<220>
   <223> specific PCR primer for MSP protein
<400> 8
   tctcttatct agagataccc 20
<210> 9
   <211> 31
   <212> DNA
   <213> Artificial Sequence
<220>
   <223> primer for PCR
<400> 9
   aaaaaagtcg acgccaaaaa ggccgagctg g 31
<210> 10
   <211> 31
   <212> DNA
   <213> Artificial Sequence
<220>
   <223> primer for PCR
<400> 10
   aaaaaagtcg acgagaagta cctcccgttc c 31
<210> 11
   <211> 31
   <212> DNA
   <213> Artificial Sequence
<220>
   <223> primer for PCR
<400> 11
   aaaaaaatcg atttaaagct ccatgcacag g 31
<210> 12
   <211> 34
   <212> DNA
   <213> Artificial Sequence
<220>
   <223> primer for PCR
<400> 12
   ggtccatatg aacgagtcca aggaaatatt atcc 34
<210> 13
   <211> 40
   <212> DNA
   <213> Artificial Sequence
<220>
   <223> primer for PCR
<400> 13
   ggacggatcc tcattagcta cagaaaactc cctcaaagag 40

## Description

### Technical Field

The present invention relates to diagnostic method and diagnostic reagents for malaria. Particularly, the present invention relates to diagnostic methods and diagnostic reagents for malaria which detects malaria-specific antibodies in blood by using antigens of malarial Protozoa. Also, the present invention relates to diagnostic methods and diagnostic reagent for malaria which detects malaria-specific IgM in blood by using antigens of malarial Protozoa.

### Background Art

Malaria is a disease caused by malarial Protozoa that infects within human erythrocytes and is carried by mosquitoes. Billion people in the world reside in malaria-risk area and over 500 million people become infected by malaria each year. Malaria causes more than 2 million death each year. Malaria widely spreads all over the world, however, in some regions, malarial infection was eradicated or decreased since 1960's due to effective controls. However, recently, incidence of malaria increases all over the world again, due to increase of drug-resistant strain, increase in resistance to insecticide, abnormal weather such as El nino, etc.

Malaria can be categorized into African type, American type and Asian type. Each type differs in geographical distribution as well as characteristics in species and genetic inclination which Protozoa exhibit.

Life cycle of malaria is divided into Schizogony and Sporogony. Schizogony is the life cycle in human host, and Sporogony is the life cycle in mosquito host. Human is infected by sporozoites by the bite of infected mosquitoes. The sporozoites transfer to human liver through blood vessels, and they exist as a dormant state or multiply and develop into schizonts in hepatic cells. After a definite time, the schizonts enter the blood circulation, and multiply in full-scale. When the schizont matures, it ruptures and releases thousands of merozoites into the blood stream. Merozoites invade erythrocytes, and most merozoites go through another round of a sexual reproduction, again forming schizonts. Some of the merozoites change into gametocytes. The gametocytes circulate in human bloodstream. When a mosquito bites the infected person, the mosquito sucks up gametocytes along with blood. Then, male gametocyte and female gametocyte in the gut of the mosquito fuse to form zygote. The zygote, which develops in the gut wall as an oocyte, eventually gives rise to the infective sporozoite, which invades the salivary glands and stomach of the mosquito. The mosquito then can infect another human host.

Drug such as chloroquine and primaquine have been used in treatment of malaria. However, recently, malaria Protozoa have developed drug resistance to such Drug. Therefore, acquired drug resistance poses a serious difficulty on treatment of malaria.

In Korea, malaria has been known as Hakjil. From the 1960s, attempts to eradicate this disease begun, and in the 1970s, cases of malaria were rarely reported. However, after a patient infected with malaria Protozoa was reported near DMZ in 1993, malaria begun to spread rapidly, and it was reported that the number of patients infected with malaria Protozoa was 3,800 in 1998.

Human malaria can be caused by one of four known parasites: *Plasmodium vivax, Plasmodium falciparum, Plasmodium malariae and Plasmodium ovale.* Among them, malaria which occurrs in Korea is caused by *Plasmodium vivax* (Choi. S.Y., Korean J Parasit., 32(4), 281-284). *Plasmodium vivax* is characterized in that diagnosis thereof is difficult because latent period is long, and number of Protozoa in blood is few. The imported diagnostic reagent for malaria detects malarial antigens such as Lactate Dehydrogenase of malaria. However, the imported diagnostic reagent has a difficulty in diagnosing malaria of Korean type where latent period is long, and numbers of Protozoa and antigen in blood are few, because sensitivity of the diagnostic reagent is only 50%.

A blood smear method has been used in the diagnosis of malaria most frequently. This method is a classical method wherein blood cells infected with malaria Protozoa are stained and the stained sample is then observed through a microscope. However, this method takes a longer time, and technical education of the examiner is required to judge blood cells infected with malaria Protozoa. Recently, a diagnostic reagent detecting malarial antigens has been commercially available. However, the concurrent use of the diagnostic reagent and the blood smear method is recommended because the sensitivity of the diagnostic reagent is low. Another method to examine genes of malaria Protozoa is Polymerase Chain Reaction method. This method has high sensitivity, however, there is a difficulty in processes thereof. Therefore, it is not put to practical use and is used only on laboratory scale. Also, such conventional diagnostic methods has a difficulty in diagnosing malaria of Korean type where latent period is long, and number of Protozoa in blood is few.

Hitherto studies on expression of malarial antigen itself have been few, and methods for obtaining proteins by overexpressing genes encoding antigen proteins have been developed. Most antigens were expressed after they were fused with proteins such as blood coagulation factor Xa (Ellinger et al., Virol. 180, 81, 1991), protein A of Staphylococcus(Marczinovits et al., J. Biochem., 31, 225, 1993) and β-galactosidase. As a result, expression amount of fused antigens increased. However, when they are used as diagnostic reagents, pseudo-positive signals increased due to the proteins that were partners of fusion. Although there are attempts to solve the above problem by means of cutting the fused proteins(Chang et al., Biotechnology 3, 985-990, 1985 ; Ellinger et al., Virology, 180, 811-813, 1991), it is not effective in additional decrease of pseudo-positive signals, and the yield decreases and the preparation cost increases because cutting and purification processes to remove the fused proteins from the antigens are complex.
Soares et al. American Journal of Tropical Medicine & Hygiene, Vol. 60, No. 3, 1999, pp 357-363, discloses a general indirect ELISA method wherein PV200c19 fusion proteins were attached to a plate, reacted with patient serum and then an immunoassay was performed by treating labelled anti-human IgG or anti-human IgM.
Tyagi P. et al., Indian Journal of Malariology, Vol. 36, No. ½, March 1999, pp. 12-18, discloses Plasmodium CICs, namely circulating immuno-complexes of IgG and IgM directed against P. vivax and P. falciparum antigens; this reference is not concerned with the detection of malaria specific antibodies in blood.
Ray K. et al., Journal of Communicable Diseases, Vol. 24, No. 2, June 1992, pp. 82-86, discloses an indirect immunofluorescence test for detecting IgM specific antibodies as serodiagnosis of malarias; an antigen derived from Plasmodium falciparum for detecting antibodies was used.
Gibson et al., Molecular and Biochemical Parasitology, Vol. 50, No. 2, 1992, pp 325-334, discloses the detection of anti-malarian antibodies using PV200 and the evaluation of the utility and diagnosis and immunoprophylaxis, focusing on the structure and expression of the gene for PV200 without mentioning a diagnostic reagent of a specific method.
Kaslow et al., Immunol. Lett., Viol. 51, July 1996, pp 187-189, discloses the PV200-19 polypeptide with 6-cistidine references focusing on the development of a vaccine candidate without mentioning detection of malaria specific antibodies and patient blood.
Gentz R. et al., EMBO Journal, Vol. 7, No. 1, 1988, pp 225-230, discloses the preparation of a monoclonal antibody specific for a variety of Plasmodia isolates using the surface antigens of 19 kDa of Plasmodium Falciparum.

### Disclosure of Invention

To solve the above-mentioned problems, the present inventors have developed a diasgnostic method for malaria by detecting malaria-specific antibodies in blood of malaria patients. More particularly, the present invention relates to a diagnostic method and diagnostic reagent for malaria which detects malaria-specific antibodies in blood by using antigens of malarial Protozoa. These assays are defined as follows:
A diagnostic method for detecting malaria-specific antibodies in blood, comprising the steps of:
   (a) immobilizing a surface antigen of a part or whole of PV200C polypeptide in the C-terminus of Merozoite Surface Protein of Plasmodium Vivax on a solid support;
   (b) pouring the blood or plasma sample;
   (c) adding labelled antigen conjugates consisting of a part or whole of PV200C polypeptide in the C-terminus of Merozoite Surface Protein of Plasmodium Vivax and marker; and
   (d) inducing colour development by using a substrate solution containing a colour fixing agent, wherein said PV200C polypeptide is the amino acid sequence as shown in SEQ ID No: 1.
A diagnostic method for detecting malaria-specific IgM in blood, comprising the steps of:
   (a) immobilising anti-human IgM antibodies on a solid support
   (b) pouring blood serum or plasma sample;
   (c) adding labelled antigen conjugates consisting of markers and antigens of a part or whole of PV200C polypeptide in the C-terminus of Merozoite Surface Protein of Plasmodium Vivax and marker; and
   (d) inducing colour development by using a substrate solution containing a colour fixing agent, wherein said PV200C polypeptide is the amino acid sequence as shown in SEQ ID No: 1.
As an another aspect, the present inventors have developed diagnostic method and diagnostic reagent for malaria which detects only malaria-specific IgM in blood of malaria patients by using antigens of malarial Protozoa.

These are defined as follows:

A diagnostic reagent for malaria detecting malaria-specific antibodies in blood, comprising:
a solid support coated with surface antigens of a part or whole of PV200C polypeptide in the C-terminus of Merozoite Surface Protein of Plasmodium Vivax;
labeled antigen conjugated consisting of a part or whole of PV200C polypeptide in the C-terminus of Merozoite Surface Protein of Plasmodium Vivax;
and markers; and
a substrate solution containing a color fixing agent, wherein said PV200C polypeptide is the amino acid sequence as shown in SEQ ID No: 1.

A diagnostic reagent for malaria detecting malaria-specific antibodies in blood, comprising:
a solid support coated with anti-human IgM antibodies;
labeled antigen conjugate consisting of markers and antigens of part or whole of PV200C polypeptide in the C-terminus of Merozoite Surface Protein of Plasmodium Vivax;
and
a substrate solution containing a color fixing agent, wherein said PV200C polypeptide is the amino acid sequence as shown in SEQ ID No: 1.

Among antibodies against malaria, IgG can last for several years or several tens of years after experience of malaria. Therefore, a method of examining antibodies including IgG against malaria in the area where malaria was prevalent for a long time results in many pseudo-positive signals, which can cause confusion. In the antigen sandwich immunoassay of the present invention, few malarial antibody-positive responses have been found in teens to thirties, however, 5% or more of malarial antibody-positive responses have been found in forties or more, in case of normal people uninfected with malaria. That is, relatively high IgG pseudo-positive signals occur in forties or more. This is because probability that people over forties were expose to mosquitoes infected with malaria before 1960s is high. Similarly, in the area where malaria is prevalent, malaria-specific IgGs remain in the body of man who completely recovers from malaria, and relatively high pseudo-positive signals appear in the assay for antibody to malaria. Therefore, effectiveness of the assay for antibody against malaria to diagnose malaria will decrease. Accordingly, for diagnosing malaria in people over forties or the area where malaria is prevalent, a method of examining IgM of which titer disappears with time after the treatment of malaria will be more effective than a method of examining IgG of which titer remains even after the treatment of malaria.

The immunoassay for malaria detecting malaria-specific IgM in blood by using antigens of malarial Protozoa according to the above described invention is more sensitive than the prior art immunoassay detecting specific antigens, and makes an early diagnosis of malaria patient possible. Also, the immunoassay for malaria detecting malaria-specific IgM according to the present invention can distinguish malaria patients from normal people who completely recover from malaria.

As another reference aspect, to overcome the above-mentioned problems of the prior art in obtaining antigen proteins and develop an immunoassay and diagnostic reagent for malaria which is suitable for diagnosing malaria of Korean type, the present inventors have developed a preparation method wherein Merozoite Surface Protein of *Plasmodium vivax*, preferably C-terminus of Merozoite Surface Protein of *Plasmodium vivax,* more preferably polypeptide PV200C is cloned from blood of domestic malaria patients, and then is expressed from yeast or E.Coli and purified without cutting.

The above preparation method can produce antigens which is very sensitive to antibody as well as highly pure in a large amount by expressing the recombinant Surface Protein of malarial Protozoa from yeast or E.Coli and then separating and purifying it in a simple method. Such produced antigens are useful in diagnostic reagents judging malaria patients by detecting antibody against malarial antigens.

In addition, such produced antigens can be used as a vaccine.

It is the first object of the present invention to provide a diagnostic method and diagnostic reagent for malaria which detects malaria-specific antibodies in blood by using antigens of malarial Protozoa as described above. It is the second object of the present invention to provide a diagnostic method using anti-human IgM antibody as an antigen of the labeled antigen conjugate.

It is a further object of the present invention to provide a diagnostic method and diagnostic reagent for malaria which detects malaria-specific IgM in blood by using antigens of malarial Protozoa. It is a further object of the present invention to provide a diagnostic method and diagnostic reagent for malaria which detects malaria-specific IgM in blood by using anti-human IgM antibody.

On a referred aspect further provided is a preparation method wherein a recombinant expression vector is prepared by means of cloning genes of Merozoite Surface Protein of *Plasmodium vivax,* and is transformed into yeast and then surface protein expressed from the yeast transformant is separated and purified to have a high purity. In a further reference aspect a method is provided of producing antigens in a large amount for diagnosing *Plasmodium vivax,* which have high sensitivity and specificity and markedly low pseudo-positive signals by means of the above-mentioned preparation method.

In a further reference aspect a preparation method wherein a recombinant expression vector is prepared is provided by means of cloning genes of Merozoite Surface Protein of *Plasmodium vivax,* and is transformed into E.Coli and then surface protein expressed from the E.Coli transformant is separated and purified to have a high purity. In a further reference aspect a method of producing antigens in a large amount for diagnosing *Plasmodium vivax* is provided which have high sensitivity and specificity and markedly low pseudo-positive signals by means of the above-mentioned preparation method.

In accordance with the present invention, there are provided a diagnostic method and diagnostic reagent for malaria detecting malaria-specific antibodies in blood as described above which are more sensitive than the prior art immunoassay detecting specific antigens, and can diagnose malarial carriers as well as malaria patients, and are useful in diagnosing malaria of Korean type where latent period is long and numbers of Protozoa and antigen in blood are few.

Also, in accordance with the present invention, there are provided a diagnostic method and diagnostic reagent for malaria detecting malaria-specific IgM in blood as described above which can distinguish malaria patients from normal people who completely recover from malaria.

Further, in a reference aspect there is provided a preparation method wherein surface antigens of malarial Protozoa with high purity can be produced more easily and rapidly than the prior art method does. The surface protein of malarial Protozoa purified by the preparation method of the present invention has high purity, sensitivity and specificity, and markedly low pseudo-positive signals, and useful in the diagnostic reagent for malaria.

### Brief Description of Drawings

Figure 1 shows a preparation method of expression vector pYLJ-MSP expressing the surface antigen of malarial Protozoa from yeast.
Figure 2 is a photograph showing a result of electrophoresis of the surface antigen purified by using Probond adsorption resin on SDS-polyacrylamide gel.
   Lane 1 is the pellet after centrifugation of the cell culture;
   Lane 2 is the supernatant that was concentrated after centrifugation of the cell culture; and
   Lane 3 is the concentrated supernatant that was purified on Probond column.
Figure 3 is a photograph showing an assay result of the antigenicity by performing Western Blotting using the serum of the malaria patient.
   Lane 4 is the cell culture that was concentrated; and
   Lane 5 is the purified Merozoite Surface Protein.
Figure 4 shows a preparation method of expression vector pELK-MSP expressing PV200C polypeptide present in C-terminus of Merozoite Surface Protein of malarial Protozoa from E.Coli.
Figure 5 is a photograph showing a result of electrophoresis of PV200C polypeptide purified by using histidine affinity resin on SDS-PAGE.
   M is a size marker;
   Lane 1 is the cell culture in which the expression was induced;
   Lane 2 is the cell culture in which the expression was not induced; and
   Lane 3 is the protein that was purified on a column.
Figure 6 is a photograph showing a result of Western Blotting of PV200C polypeptide using the serum of the malaria patient.
   M is a size marker;
   Lane 4 is the cell culture in which the expression was induced; and
   Lane 5 is the purified Merozoite Surface Protein.
Figure 7 is a graph showing a result of diagnosing 216 malaria-positive samples and 353 malaria-negative samples for malaria, according to malaria-specific IgM capture enzyme immunoassay.
Figure 8 is a graph showing results of diagnosing 75 malaria-positive samples and 92 malaria-negative samples for malaria, according to malaria-specific IgM capture enzyme immunoassay and indirect enzyme immunoassay.
Figure 9 is a graph showing results of diagnosing 129 malaria-negative samples classified by age for malaria, according to malaria-specific IgM capture enzyme immunoassay and antigen sandwich enzyme immunoassay.

### Best Mode for Carrying out the Invention

To achieve the above objects, the present invention provides a diagnostic method for malaria detecting malaria-specific antibodies in blood by using antigens of malarial Protozoa, and a diagnostic reagent for malaria detecting malaria-specific antibodies in blood, comprising antigens of malarial Protozoa, both as described above.

Also, the present invention provides a diagnostic method for malaria detecting malaria-specific IgM in blood by using antigens of malarial Protozoa, and a diagnostic reagent for malaria detecting malaria-specific IgM in blood, comprising antigens of malarial Protozoa, both as described above.

In particular, the present invention provides a diagnostic method for detecting malaria-specific antibodies in blood, comprising the steps of:
(a) immobilizing a surface antigen of a part or whole of PV200C polypeptide, in the C-terminus of Merozoite Surface Protein of Plasmodium Vivax on a solid support;
(b) pouring the blood or plasma sample;
(c) adding labelled antigen conjugates consisting of a part or whole of PV200C polypeptide, in the C-terminus of Merozoite Surface Protein of Plasmodium Vivax and marker; and
(d) inducing colour development by using a substrate solution containing a colour fixing agent, wherein said PV200C polypeptide is the amino acid sequence as shown in SEQ ID No: 1.

Further, the present invention provides a diagnostic method for detecting malaria-specific IgM in blood, comprising the steps of:
(a) immobilising anti-human IgM antibodies on a solid support
(b) pouring blood serum or plasma sample;
(c) adding labelled antigen conjugates consisting of markers and antigens of a part or whole of PV200C polypeptide in the C-terminus of Merozoite Surface Protein of Plasmodium Vivax and marker; and
(d) inducing colour development by using a substrate solution containing a colour fixing agent, wherein said PV200C polypeptide is the amino acid sequence as shown in SEQ ID No: 1.

Further, the present invention provides a diagnostic reagent for malaria detecting malaria-specific antibodies in blood, comprising:
a solid support coated with surface antigens of a part or whole of PV200C polypeptide in the C-terminus of Merozoite Surface Protein of Plasmodium Vivax;
labeled antigen conjugated consisting of a part or whole of PV200C polypeptide in the C-terminus of Merozoite Surface Protein of Plasmodium Vivax;
and markers; and
a substrate solution containing a color fixing agent, wherein said PV200C polypeptide is the amino acid sequence as shown in SEQ ID No: 1.

Further, the present invention provides a diagnostic reagent for malaria detecting malaria-specific antibodies in blood, comprising:
a solid support coated with anti-human IgM antibodies;
labeled antigen conjugate consisting of markers and antigens of part or whole of PV200C polypeptide in the C-terminus of Merozoite Surface Protein of Plasmodium Vivax;
and
a substrate solution containing a color fixing agent, wherein said PV200C polypeptide is the amino acid sequence as shown in SEQ ID No: 1.

Also, an expression vector is provided in a reference aspect comprising genes of Merozoite Surface Protein of *Plasmodium Vivax,* α-factor leader peptide of yeast and histidine residues. Further provided is a yeast transformant pYLJ-MSP/S. *cerevisiae* INVSC1 (Deposit No. KCTC 0937BP) transformed with the above expression vector. Further provided is a for preparing Merozoite Surface Protein of malarial Protozoa by using a yeast transformant. Further provided is a diagnostic reagent for malaria, comprising the surface proteins produced by the above preparation method.

Further provided is an expression vector comprising genes of Merozoite Surface Protein of *Plasmodium Vivax ,* histidine marker and T7 promotor. Further provided is E.Coli transformant pELK-MSP/BL21 (Deposit No. KCTC 0936BP) transformed with the above expression vector. Further provided is a method for preparing Merozoite Surface Protein of malarial Protozoa by using E. Coli transformant. Further provided is a diagnostic reagent for malaria, comprising the surface proteins produced by the above preparation method.

The present invention will hereinafter be described in more detail.

The present invention provides a diagnostic method for malaria detecting malaria-specific antibodies in blood by using antigens of malarial Protozoa, and a diagnostic reagent for malaria detecting malaria-specific antibodies in blood, comprising antigens of malarial Protozoa as described above. Also, the present invention provides a diagnostic method for malaria detecting malaria-specific IgM in blood by using antigens of malarial Protozoa, and a diagnostic reagent for malaria detecting malaria-specific IgM in blood, comprising antigens of malarial Protozoa, as described above.

Preferably, in the above method and diagnostic reagents of the present invention, a recombinant antigen may be used as the antigen of malarial Protozoa.

In the above method and diagnostic reagents of the present invention, a surface antigen of malarial Protozoa is used as the antigen of malarial Protozoa. This surface antigen of malarial Protozoa includes a part or whole of Merozoite Surface Protein of *Plasmodium Vivax*, namely in the C-terminus of Merozoite Surface Protein of *Plasmodium Vivax.* The above C-terminus of Merozoite Surface Protein of *Plasmodium Vivax* comprises amino acid sequences that are common to subspecies of *Plasmodium Vivax,* and especially comprises a polypeptide consisting of 108 amino acids (referred as "PV200C" hereafter) which exhibit 100% homology among subspecies of *Plasmodium Vivax,* as shown in SEQ. ID NO : 1. A preferable example of the C-terminus of Merozoite Surface Protein of *Plasmodium* Vivax includes a part or whole of PV200C polypeptide in the C-terminus of Merozoite Surface Protein of *Plasmodium Vivax.* The amino acid sequences of the PV200C polypeptide are the amino acid sequences as shown in SEQ. ID NO : 1.

The PV200C polypeptide can be expressed in a large amount from recombinant transformants. The present inventors prepared a yeast transformant and E.Coli transformant which express PV200C polypeptide, and deposited them in Korean Collection for Type Cultures of Korea Research Institute of Bioscience and Biotechnology on December 18, 1999 (Deposit No. KCTC 0937BP, KCTC 0936BP). 0936BP).

The present invention provides a method for malaria detecting malaria-specific antibodies in blood by using antigens of malarial Protozoa. Preferably, the above immunoassay detecting malaria-specific antibodies in blood according to the present invention may be an enzyme immunoassay.

Preferably, the present invention provides a diagnostic method for detecting malaria-specific antibodies in blood, comprising the steps of:
(a) immobilizing a surface antigen of a part or whole of PV200C polypeptide in the C-terminus of Merozoite Surface Protein of Plasmodium Vivax on a solid support;
(b) pouring the blood or plasma sample;
(c) adding labelled antigen conjugates consisting of a part or whole of PV200C polypeptide in the C-terminus of Merozoite Surface Protein of Plasmodium Vivax and marker; and
(d) inducing colour development by using a substrate solution containing a colour fixing agent, wherein said PV200C polypeptide is the amino acid sequence as shown in SEQ ID No: 1.

Also, as a preferable example of the above immunoassay for malaria, the present invention provides an immunoassay detecting malaria-specific antibodies in blood, comprising:
(a') expressing. Merozoite surface antigens of *Plasmodium Vivax* from transformants and purifying it;
(a) immobilizing the purified surface antigens on a solid support;
(b) pouring blood serum or plasma sample to combine malaria-specific antibodies with the surface antigens immobilized on the solid support;
(c) adding labeled antigen conjugates consisting of antigens and markers to combine the labeled antigen conjugates with the malaria-specific antibodies;
(d) inducing color development by using the markers of the labeled antigen conjugates bound to the malaria-specific antibodies; and
(e) measuring the absorbance by using a 96-well plate reader.

A preferable example of the above solid support includes wells of a well plate. Also, a preferable example of the above method may further comprise a step of washing the solid support such as well plate after the step of combining the labeled antigen conjugate with the malaria-specific antibodies. The step of analyzing by using the markers of the labeled antigen conjugates includes, for example, the step of inducing color development by using the markers of the labeled antigen conjugates bound to the malaria-specific antibodies; and the step of measuring the absorbance by using a 96-well plate reader. The step of inducing color development by using the markers of the labeled antigen conjugates includes, for example, addition of a substrate solution consisting of a buffer solution and a color fixing agent.

On one aspect an indirect immunoassay for malaria detecting malaria-specific antibodies in blood is provided using a solid support coated with antigens of malarial Protozoa, preferably surface antigens of malarial Protozoa. The above immunoassay uses anti-human IgG antibody and/or anti-human IgM antibody derived from goat combined with marker, preferably horse radish peroxidase(HRP) as a labeled antigen conjugate.

In the above aspect of indirect immunoassay for malaria malaria-specific antibodies in blood of the patient are combined with the surface antigens of malarial Protozoa immobilized on the solid support and then HRP-anti-human IgG antibody or HRP-anti-human IgM antibody which is the labeled antigen conjugate is combined with the antigen-antibody complex. Thereafter, a color fixing agent that is included in the substrate solution added is decomposed by HRP of the labeled antigen conjugate to induce a color development, and then absorbance is measured by using a 96-well plate reader to detect existence of malaria-specific antibodies and amount of the antibodies. In the above indirect immunoassay, malaria-positive samples(plasma of the malaria patients) and malaria-negative samples(plasma of normal people) were diagnosed. As a result, the above method exhibited approximately 90% of sensitivity and 95:8% of specificity.

As another aspect provided is an antigen sandwich immunoassay for malaria detecting malaria-specific antibodies in blood, using a solid support coated with antigens of malarial Protozoa, preferably surface antigens of malarial Protozoa. The above immunoassay uses a surface antigen of malarial Protozoa, preferably a part or whole of Merozoite Surface Protein of *Plasmodium Vivax* combined with marker, preferably horse radish peroxidase(HRP) as a labeled antigen conjugate.

In the aspect of antigen sandwich immunoassay for malaria , malaria-specific antibodies in blood of the patient are combined with the surface antigens of malarial Protozoa immobilized on the solid support, and then HRP-surface antigen of malarial Protozoa which is the labeled antigen conjugate is combined with the antigen-antibody complex. Thereafter, a color fixing agent that is included in the substrate solution added is decomposed by HARP of the labeled antigen conjugate to induce a color development, and then absorbance is measured by using a 96-well plate reader to detect existence of malaria-specific antibodies and amount of the antibodies. In the above antigen sandwich immunoassay, malaria-positive samples(plasma of the malaria patients) and malaria-negative samples(plasma of normal people) were diagnosed. As a result, the above method exhibited 98.5% of sensitivity and 99.6% of specificity.

The above indirect immunoassay indirectly detects malaria-specific antibodies in blood of the patient, by using anti-human IgG antibody and/or anti-human IgM antibody derived from goat as an antigen of the labeled antigen conjugate. In the meantime, the above antigen sandwich immunoassay can detect all malaria-specific antibodies in blood of the patient, by using a surface antigen itself derived from malarial Protozoa as an antigen of the labeled antigen conjugate.

The immunoassay for malaria of the present invention provides a solid support coated with antigens of malarial Protozoa. When the blood sample of man who is suspected to be a malaria patient is poured into the solid support, malaria-specific antibodies' in blood become specifically combined with the antigens immobilized on the solid support. After the labeled antigen conjugate is added to this, the labeled antigen conjugate becomes combined with malaria-specific antibodies by recognizing Fc (fragment crystallization) region of the malaria-specific antibodies combined with the antigens immobilized on the solid support. Then, the marker of the labeled antigen conjugate decomposes the color fixing agent in the substrate solution to induce a color development. Finally, the immunoassay for malaria of the present invention diagnose the infection of malaria by detecting existence of antibodies in blood and amount of the antibodies by measuring the absorbance.

The above indirect immunoassay detects malaria-specific antibodies combined with the antigen immobilized on the solid support by using marker-anti-human IgG antibody and/or marker-anti-human IgM antibody. Also, the above antigen sandwich immunoassay of the present invention detects malaria-specific antibodies combined with the antigen immobilized on the solid support by using the labeled antigen conjugate comprising the antigen of malarial Protozoa. In above two methods, plasma of the malaria patients and plasma of normal people were diagnosed for malaria. As a result, both methods exhibited more than 90 % of sensitivity and specificity.

The present invention provides a diagnostic reagent for malaria detecting malaria-specific antibodies in blood, comprising:
a solid support coated with surface antigens of a part or whole of PV200C polypeptide in the C-terminus of Merozoite Surface Protein of Plasmodium Vivax;
labeled antigen conjugated consisting of a part or whole of PV200C polypeptide in the C-terminus of Merozoite Surface Protein of Plasmodium Vivax;
and markers; and
a substrate solution containing a color fixing agent, wherein said PV200C polypeptide is the amino acid sequence as shown in SEQ ID No: 1.

The surface antigen of malarial Protozoa is a part or whole of Merozoite Surface Protein of *Plasmodium Vivax*, namely includes C-terminus of Merozoite Surface Protein of *Plasmodium vivax*, i.e. a part or whole of PV200C polypeptide in the C-terminus of Merozoite Surface Protein of *Plasmodium Vivax*. The amino acid sequences of the PV200C polypeptide are amino acid sequences as shown in SEQ. ID NO : 1.

Preferably, in the above diagnostic reagent, the present invention may utilize anti-human anti-human IgM antibody as the antigen of the labeled antigen conjugate.

Preferably, in the above diagnostic reagent, the present invention may utilize a surface antigen of malarial Protozoa as the antigen of the labeled antigen conjugate. The surface antigen of malarial Protozoa includes a part or whole of Merozoite Surface Protein of *Plasmodium Vivax*, i.e. C-terminus of Merozoite Surface Protein of *Plasmodium Vivax*, *i*.e. a part or whole of PV200C polypeptide in the C-terminus of Merozoite Surface Protein of *Plasmodium Vivax*. The amino acid sequences of the PV200C polypeptide are amino acid sequences as shown in SEQ. ID NO : 1.

Preferably, the present invention may utilize an enzyme such as horse radish peroxidase(HRP) and alkaline phosphatase, colloidal gold, fluorescent materials, dyes or the like as the marker of the labeled antigen conjugate. More preferably, the present invention may utilize horse radish peroxidase(HRP) as the marker of the labeled antigen conjugate.

In the above diagnostic reagent of the present invention, the labeled antigen conjugate detects existence of malaria-specific antibodies by binding to Fc (fragment crystallization) region of the malaria-specific antibodies in which there is little variation.

As a preferable labeled antigen conjugate, the present invention provides HRP-anti-human IgM antibody wherein HRP-anti-human IgM antibody is derived from goat. Also, as a preferable labeled antigen conjugate, the present invention provides HRP-surface antigen conjugate wherein the surface antigen is a part or whole of surface protein of Malaria Protozoa as defined above.

The diagnostic reagent of the present invention comprises a substrate solution inducing color development when reacted with the marker of the labeled antigen conjugate. Preferably, the substrate solution consists of a buffer solution and a color fixing agent which induces color development when reacted with the marker of the labeled antigen conjugate. A preferable example of the color fixing agent includes tetramethylbenzidine and the like. The color fixing agent such as tetramethylbenzidine in the substrate solution is decomposed by HRP which is a preferable marker of the labeled antigen conjugate to induce a color development. Then, existence of malaria-specific antibodies and amount of the antibodies are detected by measuring the absorbance.

The present invention provides an immunoassay for malaria detecting malaria-specific IgM in blood by using antigens of malarial Protozoa. Preferably, the present invention provides an IgM capture immunoassay detecting malaria-specific IgM in blood by using antigens of malarial Protozoa, and more preferably, the present invention provides an IgM capture enzyme immunoassay detecting malaria-specific IgM in blood by using antigens of malarial Protozoa. Also, preferably, the present invention provides an immunoassay detecting malaria-specific IgM in blood by using antigens of malarial Protozoa and anti-human IgM antibody.

The present invention provides a 96-well plate coated with anti-human IgM antibody derived from goat as a preferable solid support for diagnosing malaria. Also, the present invention provides a labeled antigen conjugate consisting of a marker and an antigen of malarial Protozoa to detect malaria-specific IgM. The labeled antigen conjugate of the present invention consists of a marker and an antigen, and the antigen maybe preferably a recombinant surface antigen of malarial Protozoa purified from yeast or E.Coli transformant to detect malaria-specific IgM. The marker of the labeled antigen conjugate includes, but is not limited to, for example, an enzyme such as horse radish peroxidase(HRP) and alkaline phosphatase, colloidal gold, fluorescent materials, dyes or the like. Preferably, the marker of the labeled antigen conjugate is an enzyme, and more preferably, horse radish peroxidase(HRP).

A preferable example of IgM capture immunoassay for malaria detecting malaria-specific IgM in blood according to the present invention, comprises (i) immobilizing anti-human IgM antibodies on a solid support; (ii) pouring blood serum or plasma sample to combine malaria-specific IgM with the anti-human IgM antibodies immobilized on the solid support; (iii) adding labeled antigen conjugates consisting of markers and antigens of malarial Protozoa as defined above to combine the labeled antigen conjugates with the malaria-specific IgM; and (iv) analyzing by using the markers of the labeled antigen conjugates bound to the malaria-specific IgM.

Preferably, in the above IgM capture immunoassay, the present invention utilizes a surface antigen of malarial Protozoa as the antigen of the labeled antigen conjugate in the step (iii), which includes a part or whole of PV200C polypeptide in the C-terminus of Merozoite Surface Protein of *Plasmodium Vivax*. Also, the amino acid sequences of the PV200C polypeptide are amino acid sequences as shown in SEQ. ID NO : 1.

A more preferable example of IgM capture immunoassay, for malaria detecting malaria-specific IgM in blood according to the present invention, comprises (1) pouring blood serum or plasma sample into each well of the well plate coated with anti-human IgM antibody derived from goat; (2) washing the well plate; (3) adding the labeled antigen conjugate consisting of HRP and the recombinant surface antigen to combine the labeled antigen conjugate with the IgM bound to the anti-human IgM antibody; (4) washing the well plate; (5) adding a substrate solution consisting of a buffer solution and a color fixing agent to induce color development; and (6) measuring the absorbance by using a 96-well plate reader.

As a preferable embodiment, the immunoassay for malaria of the present invention provides a solid support coated with anti-human IgM antibody derived from goat. Also, as a preferable embodiment, the immunoassay for malaria of the present invention provides a labeled antigen conjugate wherein an antigen protein is bound to HRP as described in the example 3-3 below. The antigen protein is expressed from the recombinant yeast or E.Coli which is transformed with the expression vector prepared by insertion of genes encoding antigen proteins of *Plasmodium vivax,* and purified.

When the blood sample of man who is suspected to be a malaria patient is poured into the solid support coated with anti-human IgM antibody derived from goat, human IgM in blood becomes combined with the anti-human IgM antibody immobilized on the solid support. After the labeled antigen conjugate consisting of HRP and surface antigen of malarial Protozoa is added to the solid support, the conjugate becomes combined with IgM of the malaria patient among IgMs bound to the anti-human IgM antibodies immobilized on the solid support. The HRP of the conjugate bounded to IgM of the malaria patient decomposes the color fixing agent in the substrate solution to induce color development. Finally, the immunoassay for malaria of the present invention diagnoses the infection of malaria by detecting existence of the IgM in blood and amount of the IgM by measuring the absorbance.

In the above immunoassay of the present invention, plasmas of the malaria patients and plasmas of normal people were diagnosed for malaria. As a result, the above method exhibited more than 98% of sensitivity and more than 99% of specificity. Particularly, all the plasmas of normal people in forties or more who were infected with malaria in the past and are suspected to have IgG against malaria were diagnosed to be negative.

The present invention provides a diagnostic reagent for malaria detecting malaria-specific IgM in blood, comprising antigens of malarial Protozoa. Preferably, the present invention provides a diagnostic reagent for malaria detecting malaria-specific IgM in blood, comprising:
a solid support coated with anti-human IgM antibodies;
labeled antigen conjugate consisting of markers and antigens of part or whole of PV200C polypeptide in the C-terminus of Merozoite Surface Protein of Plasmodium Vivax;
and
a substrate solution containing a color fixing agent, wherein said PV200C polypeptide is the amino acid sequence as shown in SEQ ID No: 1.

Preferably, in the above diagnostic reagent, the present invention utilizes a part or whole of PV200C polypeptide in the C-terminus of Merozoite Surface Protein of *Plasmodium Vivax.* Also, the amino acid sequences of the PV200C polypeptide are amino acid sequences as shown in SEQ. ID NO : 1.

Anti-human IgM antibody for the diagnostic reagent of the present invention includes, but is not limited to, for example, anti-human IgM antibody derived from goat. Preferably, the anti-human IgM antibody is specific to mu chain of IgM, purified by affinity between antigen and antibody, and does not cross-react with IgG or other immunoglobulin.

The marker of the labeled antigen conjugate for the diagnostic reagent of the present invention includes, but is not limited to, for example, an enzyme such as horse radish peroxidase(HRP) and alkaline phosphatase, colloidal gold, fluorescent materials, dyes or the like. Preferably, the marker of the labeled antigen conjugate is horse radish peroxidase(HRP).

The diagnostic reagent of the present invention comprises a substrate solution inducing color development when reacted with the marker of the labeled antigen conjugate. Preferably, the substrate solution consists of a buffer solution and a color fixing agent which induces color development when reacted with the marker of the labeled antigen conjugate. A preferable example of the color fixing agent includes tetramethylbenzidine and the like. The color fixing agent such as tetramethylbenzidine in the substrate solution is decomposed by HRP which is a preferable marker of the labeled antigen conjugate to induce color development. Then, existence of the IgM and amount of the IgM are detected by measuring the absorbance.

An expression vector is provided comprising genes of Merozoite Surface Protein of *Plasmodium Vivax,* α-factor leader peptide of yeast and histidine residues. Preferably, the genes of Merozoite Surface Protein in the expression vector may comprise a part or whole of genes of Merozoite Surface Protein of *Plasmodium Vivax.* The genes of PV200C polypeptide present in the C-terminus of Merozoite Surface Protein of *Plasmodium Vivax* are preferable as the part or whole of genes of Merozoite Surface Protein of *Plasmodium Vivax.* PV200C polypeptide is known to have amino acid sequences that are common to several subspecies of *Plasmodium Vivax.* More preferably, in the expression vector, amino acid sequences of the PV200C polypeptide are amino acid sequences as shown in SEQ. ID NO : 1.

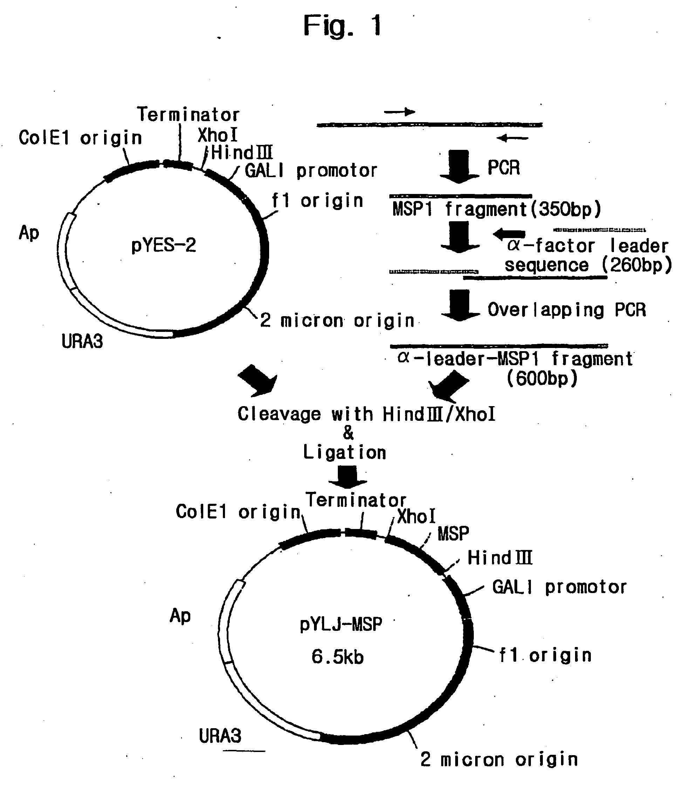

Preferably, provided is an expression vector pYLJ-MSP having a cloning map (or restriction enzyme site map) as shown in Figure 1. Also a yeast transformant pYLJ-MSP/S. *cerevisiae* INVSC1 (Deposit No. KCTC 0937BP) transformed with the above expression vector is provided. pYLJ-MSP comprises α-factor leader peptide of yeast linked with N-terminus of PV200C polypeptide, PV200C polypeptide and six histidine residues linked with C-terminus of PV200C polypeptide.

To clone the PV200C polypeptide, the present inventors purified RNA from malaria-positive blood, and prepared cDNA from the above RNA. Thereafter, the present inventors obtained amplified DNA fragments of PV200C by performing polymerase chain reaction(PCR) by using a pair of primers specific to PV200C and using the above cDNAs as a template.

In the meantime, to obtain proteins glycosylated in an active form by making PV200C polypeptide expressed in the yeast to be secreted into out of cell, α-factor leader peptide sequence of yeast was linked with N-terminus of PV200C polypeptide. Amplified DNA fragments of the α-factor leader peptide sequence of yeast could be obtained by performing polymerase chain reaction(PCR) using plasmids containing the α-factor leader peptide sequence of yeast.

To link the DNA fragments of the α-factor leader peptide of yeast with genes of PV200C polypeptide obtained as described above, an overlapping PCR was performed using the two PCR products as templates. As a result, it was found that the genes consisted of genes encoding 108 amino acids, as shown in SEQ. ID NO : 1. The present inventors obtained pYLJ-MSP which is a recombinant expression vector by inserting fragments of the genes into pYES-2 vector (refer to Figure 1). Also, the present inventors obtained a transformant pYLJ-MSP/S. *cerevisiae* INVSC1 by transforming a yeast with the recombinant expression vector pYLJ-MSP, and deposited the transformant in Korean Collection for Type Cultures of Korea Research Institute of Bioscience and Biotechnology deposited on December 18, 1999 (Deposit No. KCTC 0937BP).

Also provided is method for preparing Merozoite Surface Protein of malarial Protozoa by using a yeast transformant. Further provided is a diagnostic reagent for malaria, comprising the surface proteins produced by the preparation method. Preferably, provided is a method for preparing Merozoite Surface Protein of malarial Protozoa by using a yeast transformant, comprising (i) preparing an expression vector of Merozoite Surface Protein of malarial Protozoa; (ii) transforming the expression vector into a yeast to obtain a yeast transformant; (iii) culturing the yeast transformant to obtain surface proteins; and (iv) separating and purifying the surface proteins. More preferably, in the above preparation method, the present invention may use a column having an affinity for histidine and gel filtration chromatography as the step (iv). Preferably, the Merozoite Surface Protein of malarial Protozoa may be PV200C polypeptide in an active form. The PV200C polypeptide expressed from the above transformant was secreted into out of cell by the α-factor leader peptide coupled with N-terminus of PV200C. After secretion, the α-factor leader peptide was removed by peptidase existing in the cell membrane. Accordingly, it was confirmed that only the surface protein was secreted into culture medium.

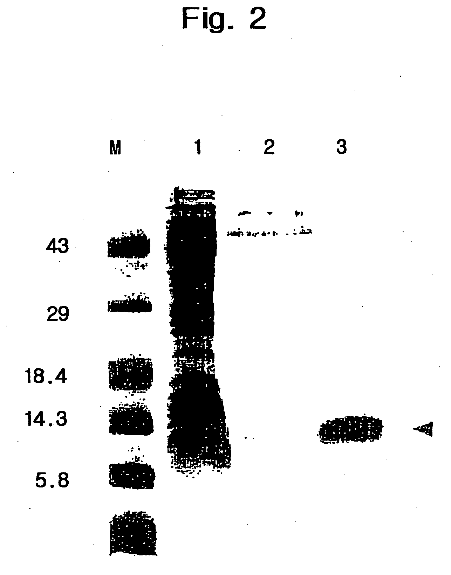

Because the surface protein expressed from the yeast transformant was secreted into culture medium, the present inventors separated the surface protein by adsorbing the surface protein on Probond column by utilizing six histidine residues linked with C-terminus of the surface protein. Then, Merozoite Surface Protein having 99% or more of high purity was obtained by purifying through a gel filtration chromatography(refer to Figure 2). The separated and purified Merozoite Surface Protein has approximately 18 KDa, and it was confirmed that the expressed surface protein was PV200C polypeptide of malarial Protozoa by analysis of amino acid sequences in N-terminus of the surface protein.

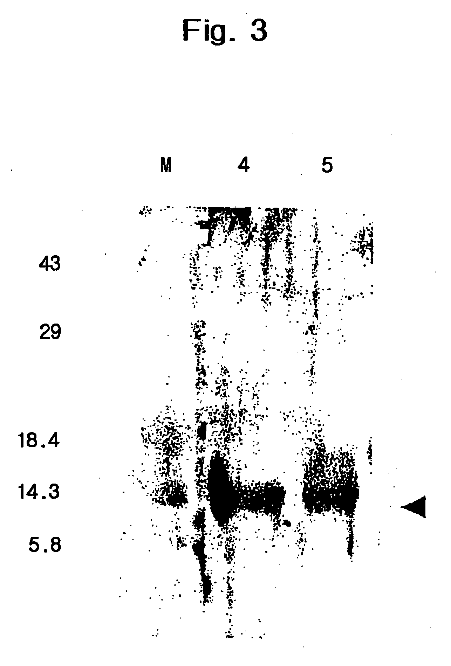

In the meantime. Western Blotting was performed with the serum of malaria patients to assay antigenicity of the surface protein separated and purified from the yeast transformant. As a result, only MSP PV200C polypeptide having 18 KDa was detected(refer to Figure 3). Therefore, it was proved that the protein separated and purified from the yeast transformant according to the preparation method of the present invention was an antigen having high sensitivity and specificity.

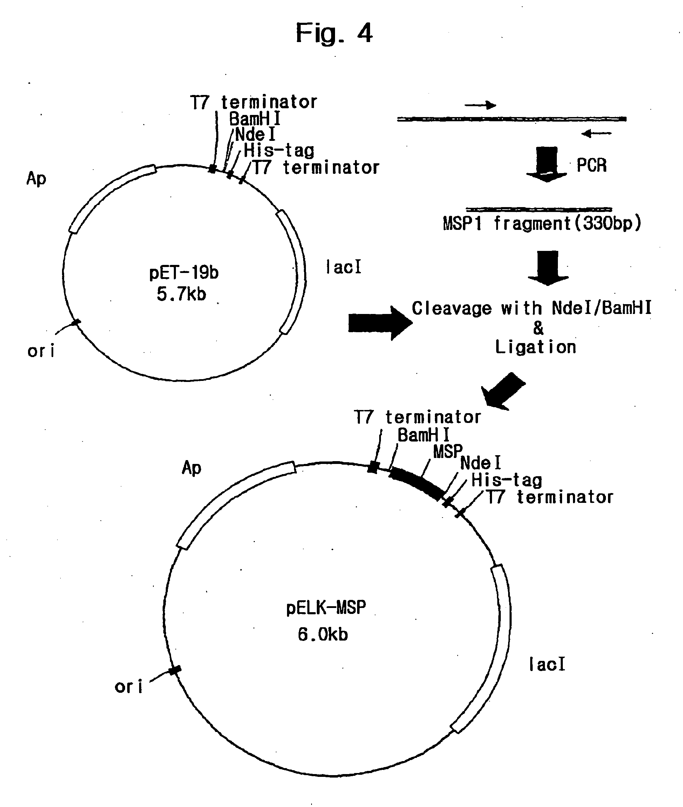

Provided in an expression vector comprising genes of Merozoite Surface Protein of *Plasmodium Vivax,* histidine marker and T7 promotor. Preferably, the genes of Merozoite Surface Protein in the expression vector of the present invention may comprise a part or whole of genes of Merozoite Surface Protein of *Plasmodium Vivax.* The genes of PV200C polypeptide in the C-terminus of Merozoite Surface Protein of *Plasmodium Vivax* are preferable as the part or whole of genes of Merozoite Surface Protein of *Plasmodium Vivax.* More preferably, in the expression vector, amino acid sequences of the PV200C polypeptide are amino acid sequences as shown in SEQ. ID NO : 1. Preferably, provided is an expression vector pELK-MSP having a cleavage map as shown in Fig. 4. Also provided is E. Coli transformant pELK-MSP/BL21 (Deposit No. KCTC 0936BP) transformed with the above expression vector. In a preferable embodiment, the present invention utilizes genes having a size of approximately 330 bp, which are obtained by polymerase chain reaction(PCR) from blood of malaria patients. It is apparent to those skilled in the art that size of genes to be inserted into adventitious gene insertion section of the expression vector, base pair sequence and the like may be varied by prior art techniques.

To obtain the expression vector which can produce the PV200C polypeptide in a large amount from E.Coli, pELK-MSP is prepared by cloning genes of PV200C in downstream of T7 promotor of pET-19b(Refer to Figure 4). Because there are lacZ genes in pET-19b that is a starting vector, when isopropyl thio-β-D-galactoside (IPTG) is added, IPTG acts as an expression introducer and induce the expression of lacZ. Therefore, the expression of PV200C from T7 promotor becomes induced.

Also, 10 histidine residues which do not make pseudo-positive signals are expressed in a state where they are fused with N-terminus of PV200C polypeptide expressed by the expression vector. Therefore, the PV200C polypeptide expressed by the expression vector is characterized in that it can be easily purified by histidine affinity resin. When the recombinant proteins were expressed in E.Coli, it was confirmed that the yield was 15~20% of the total proteins.

Further provided is E. Coli transformed with the pELK-MSP. The E. Coli transformant can be prepared by introducing pELK-MSP into E.Coli strain, BL21(DE3)lysS. The introduction method may be a prior art method such as heat shock method and electroporation. Besides BL21(DE3)lysS, various E.Coli strains for transformation can be used.

E.Coli/BL21(DE3)lysS/pELK-MSP was deposited in Korean Collection for Type Cultures of Korea Research Institute of Bioscience and Biotechnology deposited on December 18, 1999 (Deposit No. KCTC 0936BP).

Also provided is a method for preparing Merozoite Surface Protein of malarial Protozoa by using E.Coli transformant. Further provided is a diagnostic reagent for malaria, comprising the surface proteins produced by the preparation method.

Preferably provided is a method for preparing Merozoite Surface Protein of malarial Protozoa by using E. Coli transformant, comprising (i) preparing an expression vector of Merozoite Surface Protein of malarial Protozoa; (ii) transforming the expression vector into E. Coli to obtain E. Coli transformant; (iii) culturing the E. Coli transformant to obtain surface proteins; and (iv) separating and purifying the surface proteins. More preferably, in the above preparation method, the present aspect may use a column having an affinity for histidine and gel filtration chromatography as the step (iv). Preferably, the Merozoite Surface Protein of malarial Protozoa may be PV200C polypeptide in an active form. Because 10 histidine residues are expressed in a state where they are linked with N-terminus of PV200C polypeptide expressed by the expression vector, the PV200C polypeptide expressed by the expression vector is firstly purified by histidine affinity resin. After the separated protein is concentrated, the protein is secondly purified by continuously performing gel filtration chromatograph. It was confirmed that antigens of malarial Protozoa having 99% or more of high purity can be obtained by the above method.

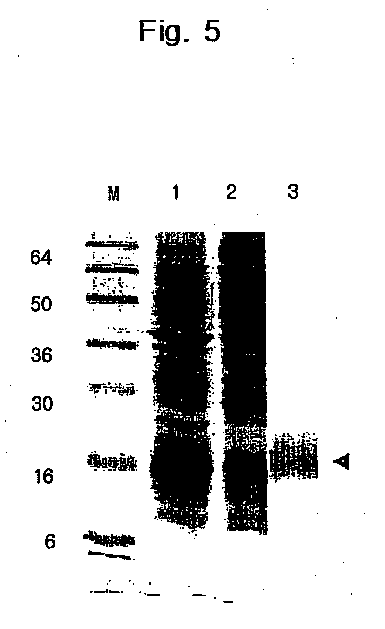

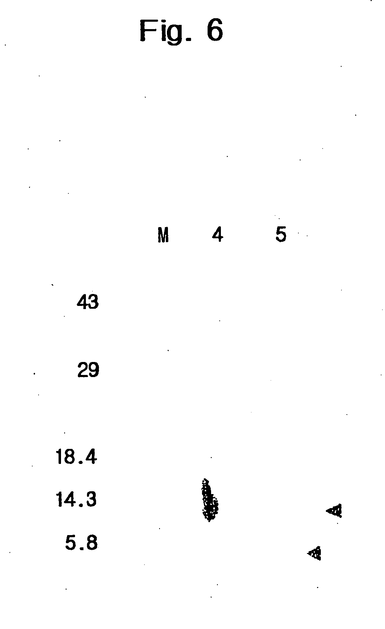

The PV200C protein obtained by the above purification method consists of 108 amino acids, has a size of 15 KDa and polypeptides that are not severed(refer to Figure 5). Antigenicity and sensitivity of the above antigen were examined using the serum of malaria patient As a result, it was confirmed by Western Blotting that the malaria patient had antibodies against PV200C and the sensitivity thereof was very high. Therefore, the above antigen can be used in a diagnostic reagent(refer to Figure 6).

The previously described versions of the present invention includes many advantages as follows.

The method and diagnostic reagent for malaria detecting malaria-specific antibodies in blood according to the present invention are more sensitive than the prior art immunoassay detecting specific antigens, and can diagnose malarial carriers as well as malaria patients, and are useful in diagnosing malaria of Korean type where latent period is long numbers of Protozoa and the antigen in blood are few, and the probability of recurrence is high.

The indirect immunoassay using anti-human IgG antibody and/or anti-human IgM antibody and the antigen sandwich immunoassay using the labeled antigen conjugate consisting of the marker and surface antigen, which are embodiments of the present invention, can diagnose even malarial carriers with high sensitivity, because they are methods detecting malarial-specific antibodies. Also, the indirect immunoassay and the antigen sandwich immunoassay of the present invention can make analysis simple, because blood samples and the labeled antigen conjugates were reacted simultaneously.

Further, the diagnostic reagent for malaria according to the present invention, comprising a solid support coated with surface antigens of malarial Protozoa; labeled antigen conjugates consisting of antigens and markers; and a substrate solution containing a color fixing agent, can detect existence of malaria-specific antibodies in blood and amount of the antibodies more simply and precisely.

The method and diagnostic reagent for malaria detecting malaria-specific IgM in blood by using antigens of malarial Protozoa according to the present invention can distinguish malaria patients from normal people who completely recover from malaria, because they detect only malaria-specific IgM in blood. Therefore, malaria can be diagnosed effectively in older people and in the area where malaria was prevalent for a long time.

Also, the method and diagnostic reagent for malaria detecting malaria-specific IgM in blood according to the present invention are very effective in diagnosing malaria of Korean type where latent period is long, numbers of Protozoa and the antigen in blood are few, and the probability of recurrence is high, because they can diagnose malaria in a latent period. Further, the method and diagnostic reagent for malaria detecting malaria-specific IgM in blood according to the present invention are useful in an early diagnosis of the malaria patient.

According to the preparation method, the Merozoite Surface Protein of malarial Protozoa with high purity can be prepared more easily and rapidly than prior art techniques by expressing MSP of malarial Protozoa in an active form from a yeast transformant using recombinant DNA technology. The surface protein purified by said preparation method has high sensitivity and specificity to antibody as well as high purity. Also, the surface protein purified by the preparation method of the present invention has markedly low pseudo-positive signals, and can be used in a diagnostic reagent and a vaccine for malaria.

Further, according to the preparation method the Merozoite Surface Protein of malarial Protozoa with high purity can be prepared more easily and rapidly than prior art techniques by expressing MSP of malarial Protozoa from E. Coli transformant as well as be produced in a large amount.

The surface protein purified by said preparation method has high sensitivity and specificity to antibody as well as high purity. Also, the surface protein purified by the preparation method has markedly low pseudo-positive signals, and can be used in a diagnostic reagent for malaria.

The invention will be further illustrated by the following examples. It will be apparent to those skilled in the art that these examples are given only to illustrate the present invention in more detail, but the invention is not limited to the examples given.

### Example 1-1. Preparation of cDNA from malaria-positive blood

TRI reagent TM (Sigma Co.) was used to purify RNA from malaria-positive blood. The purification method is the following method provided by Sigma Co. A mixture of 0.1 ml of TRI reagent TM and 0.1 ml of malaria-positive blood was left for 5 minutes at ambient temperature. 20 µl of BCP was mixed with the mixture, and the resulting mixture was left for 5 minutes at ambient temperature and then was centrifuged for 15 minutes at 12,000 rpm and 4°C. As a result of centrifugation, three layers were formed. Only the upper layer in which RNAs were contained was transferred to a new tube, and then 50 µl of isopropanol was added to the tube. The tube was left for 5 minutes at ambient temperature. The resulting mixture was centrifuged for 10 minutes at 12,000 rpm and 4°C. The supernatant was discarded. The pellet was washed with 75% ethanol and dissolved in 20 µl of deionized distilled water, which was used to prepare cDNA.

9 µl of the above-purified RNA and 1 µl of N6 random primer (Genotech) were mixed and reacted for 5 minutes at 65°C and then transferred to ice for cooling. 4 µl of RT buffer, 2 µl of 0.1M DTT(DL-Dithiothreitol)(Sigma, Cal.No. D5545), 1 µl of 10mM dNTP, 1 µl of Reverse Transcriptase(Gibco Co.), 1 µl of RNase inhibitor(Promega) and 1 µl of deionized distilled water were added to the above mixture, and the resulting mixture was reacted for 1 hour at 42°C and then reacted for 10 minutes at 70°C to prepare cDNA.

### Example 1-2. preparation of recombinant plasmids comprising genes encoding PV200C.

Polymerase chain reaction was performed using cDNAs prepared in Example 1-1 as a template and a pair of primers, SEQ. ID. NO:2 and SEQ. ID. NO:4, as follows.

The reaction mixture containing 5 µl of PCR buffer, 5 µl of 2.5 mM dNTP, 1 µl of sense primer, 1 µl of anti-sense primer, 2 µl of template of cDNA and 35 µl of deionized distilled water was reacted for 30 seconds at 94°C. Thereafter, 1 µl of Vent polymerase(BioLab) was added to the mixture. Then, the reaction having the following cycle was repeated 36 times:
- Denaturation: 94°C, 30 sec
- Primer annealing: 55°C, 30 sec
- Extension: 72°C, 30 sec

Again, polymerase chain reaction was performed using the above-amplified DNAs as a template and a pair of primers, SEQ. ID. NO:3 and SEQ. ID. NO:4. The PCR was performed in the same condition as the above condition. The amplified DNAs were confirmed through electrophoresis in 1% agarose gel. The amplifed DNAs (referred as "Pv200-ct657" hereafter) and pBluscript KS(+)(Stratagene) vector were cleavaged with restriction enzyme, EcoR V, and Pv200-ct657 was inserted into pBluscript KS(+) vector by using T4 DNA ligase(Promega). As a result, recombinant plasmids(referred as "pBC-Pv200-ct657" hereafter) were produced. Then, E. coli strain JM 109 was transformed with pBC-Pv200-ct657.

### Example 1-3. preparation of expression vector pYLJ-MSP

Polymerase chain reaction was performed using pBC-Pv200-ct657 prepared in Example 1-2 as a template and a pair of primers, SEQ. ID. NO:5 and SEQ. ID. NO:6. The PCR was performed in the same condition as the above reaction condition. The amplified DNAs(referred as "Pv200-19" hereafter) were confirmed through electrophoresis in 1% agarose gel.

In order to obtain α-factor leader peptide sequence of yeast to be linked with N-terminus of PV200C polypeptide, PCR was performed using the α-IFN pYLBC(deposit No. KCTC 0051BP) comprising the sequence encoding α-factor leader peptide of yeast as a template and a pair of primers, SEQ. ID. NO:7 and SEQ. ID. NO: 8. The PCR was performed in the same condition as the above reaction condition. The amplified DNAs(referred as "α-leader of yeast" hereafter) were confirmed through electrophoresis in 1% agarose gel.

To link the above-amplified Pv200-19 with α-factor leader peptide sequence of yeast, an overlapping PCR was performed using the above two PCR products as templates and a pair of primers, SEQ. ID. NO:6 and SEQ. ID. NO:7. A reaction mixture containing 5 µl of PCR buffer, 5 µl of 2.5 mM dNTP, 1 µl of sense primer, 1 µl of anti-sense primer, 1 µl of PCR products of Pv200-19, 1 µl of PCR products of α-factor leader peptide of yeast and 35 µl of deionized distilled water, was reacted for 30 seconds at 94°C. Thereafter, 1 µl of Vent polymerase(BioLab) was added to the mixture. Then, the reaction having the following cycle was repeated 36 times:
- Denaturation: 94°C, 30 sec
- Primer annealing: 55°C, 30 sec
- Extension: 72°C, 30 sec

The amplified DNAs(referred as "α-Pv200-19" hereafter) were confirmed through electrophoresis in 1% agarose gel.

The above-amplified α-Pv200-19 and pYES-2 vectors were cleavaged with restriction enzymes, HindIII and Xho I, respectively. Then, α-Pv200-19 and pYES-2 vector were linked by T4 ligase to be a recombinant expression vector that is referred as "pYLJ-MSP"(Refer to Figure 1). As a result of analyzing α-Pv200-19 sequence in pYLJ-MSP, it was confirmed that α-Pv200-19 consisted of the genes encoding 108 amino acids as shown SEQ. ID. NO:1. Yeast *Saccharomyces cerevisiae* INVSC1 was transformed with pYLJ-MSP according to Hinnen method (Proc. Natl. Acad Sci., U.S.A 75, 1929-1933, 1978), and then the yeast transformants were cultivated overnight in minimal medium lacking uracil. The yeast transformants were deposited in Korean Collection for Type Cultures of Korea Research Institute of Bioscience and Biotechnology deposited on December 18, 1999 (Deposit No. KCTC 0937BP)

### Example 1-4. expression and purification of MSP from yeast

In order to produce the surface protein MSP from yeast, the transformants pYLJ-MSP/S. *cerevisiae* INVSC1 were inoculated in 100 ml of YEPD medium containing 2% glucose and cultivated overnight. Then, the culture medium was transferred to 2L of YEP medium containing 1% glucose and 1% galactose, respectively, and cultivated for 72 hours at 30°C. The yeast transformants expressed PV200C polypeptide, exhausting glucose. The expressed PV200C polypeptide was secreted into out of cell by the α-factor leader peptide linked with N-terminus of PV200C polypeptide. Thereafter, the α-factor leader peptide was removed by peptidase existing in cell membrane. Accordingly, only the PV200C polypeptide was secreted into culture medium.

In order to obtain the PV200C polypeptide secreted into culture medium, the biomass was removed by centrifuging the culture medium that was cultivated for 2 days. Then, only the fluid of culture medium was taken and concentrated by ultrafiltrating apparatus(Amicon, U.S.A.). For easily purifying the concentrated fluid, the surface proteins linked with cationic histidine residues were adsorbed on Probond column(Invitrogen) to which anionic nickels were coupled by using six histidine residues linked with C-terminus of the above-amplified Pv200-19.

The contaminants which were not adsorbed on column were removed by phosphate buffer containing 10mM imidazol, and then the surface proteins adsorbed on column were desorbed and separated by 500 mM imidazol and phosphate buffer. As shown in Figure 2, the surface proteins having approximately 90% or more of purity were separated by the process. Then, the surface proteins having approximately 99% or more of high purity were obtained by concentrating the separated solution and performing Sephacryl S-200 gel filtration chromatography with the concentrate.

### Example 1-5. assay of antigenicity of the surface protein of malarial Protozoa purified from yeast

The surface protein PV200C polypeptide obtained in Example 1-4 was analyzed with 12% SDS-PAGE. As a result, it was detected in 18 kDa position, and it was identified as the object protein of the present invention by sequencing amino acids of N-terminus thereof.

In addition, Western Blotting was performed with the serum of the malaria patient to assay antigenicity of the surface protein of malarial Protozoa purified from yeast. Western Blotting was performed according to the method suggested by Towbin et al. (Proc. Natl. Acad. Sci., USA, 76, 4350-4354, 1979). After the electrophoresed gel was immersed in a buffer solution containing 25 mM Tris buffer, 190 mM glycine(pH 8.3) and 20% methanol for 30 minutes, the protein was transferred to nitrocellulose membrane by electrophoresis using the same buffer. Then, the protein-bound nitrocellulose membrane was incubated for 1 hour at ambient temperature in a phosphate buffer(pH 7.4) containing 3% Skim milk. The serum of the malaria patient diluted to 1/100 was added to the membrane and was incubated for 1 hour at ambient temperature. Thereafter, the nitrocellulose membrane was washed 5 times at 5 minute-intervals with 0.05% Tween 20/phosphate buffer solution, and then the second antibody(Vector Labs) to which HRP was conjugated was diluted to 1/2000, added to the membrane and incubated for 1 hour at ambient temperature. After the incubation, the nitrocellulose membrane was washed with the above solution, and then both 4-chloro-naphthol and hydrogen peroxide were added to the membrane to induce color development. As a result, it was confirmed that only the PV200C polypeptide was detected at 18 kDa position. Therefore, it was confirmed that the surface protein PV200C polypeptide expressed and purified by the preparation method of the present invention was an antigen having high specificity(refer to Figure 3).

### Example 2-1. Preparation of cDNA from malaria-positive blood.

TRI reagent TM(Sigma Co.) was used to purify RNA from malaria-positive blood. The purification method is the following method provided by Sigma Co. A mixture of 0.1 ml of TRI reagent TM(Sigma Co., Cat. No. T9424) and 0.1 ml of malaria-positive blood was left for 5 minutes at ambient temperature. 20 µl of BCP was mixed with the mixture, and the resulting mixture was left for 5 minutes at ambient temperature and then was centrifuged for 15 minutes at 12,000 rpm and 4°C. As a result of centrifugation, three layers were formed. Only the top layer in which RNAs were contained was transferred to a new tube, and then 50 µl off isopropanol was added to the tube. The tube was left for 5 minutes at ambient temperature. The resulting mixture was centrifuged for 10 minutes at 12,000 rpm and 4°C. The supernatant was discarded. The pellet was washed with 75% ethanol and dissolved in 20 µl of deionized distilled water treated with DEPC(Diethyl pyrocarbonate), which was used to prepare cDNA.

9 µl of the above-purified RNA and 1 µl of N6 random primer (Genotech) were mixed and reacted for 5 minutes at 65°C and then transferred to ice. 4 µl of PCR buffer, 2 µl of 0.1M DTT(DL-Dithiothreitol)(Sigma, Cal No. D5545), 1 µl of 10mM dNTP, 1 µl of Reverse Transcriptase(Gibco Co.), 1 µl of RNase inhibitor(Promega) and 1 µl of deionized distilled water were added to the above mixture, and the resulting mixture was reacted for 1 hour at 42°C and then reacted for 10 minutes at 70°C to prepare cDNA.

### Example 2-2. preparation of recombinant plasmids comprising genes encoding PV200C

Polymerase chain reaction was performed using cDNAs prepared in Example 2-1 as a template and a pair of primers, sense primer and anti-sense primer, having SEQ. ID. NO: 9 and SEQ. ID. NO:10, respectively.

The reaction mixture containing 5 µl of PCR buffer, 5 µl of 2.5 mM dNTP, 1 µl of sense primer, 1 µl of anti-sense primer, 2 µl of template of cDNA and 35 µl of deionized distilled water was reacted for 30 seconds at 94°C. Thereafter, 1 µl of Vent polymerase(BioLab) was added to the mixture. Then, the reaction having the following cycle was repeated 36 times:
- Denaturation: 94°C, 30 sec
- Primer annealing: 55°C, 30 sec
- Extension: 72°C, 30 sec.

Again, polymerase chain reaction was performed using the above-amplified DNAs as a template and a pair of primers, sense primer and anti-sense primer, having SEQ. ID. NO:10 and SEQ. ID. NO:11, respectively. The PCR was performed in the same condition as the above condition. It was confirmed that the amplified DNA had a size of approximately 657 bp through electrophoresis in 1 % agarose gel.

The amplifed DNAs(referred as "Pv200-ct657" hereafter) and pBluscript KS(+)(Stratagene) vector were cleavaged with restriction enzyme, EcoR V, and Pv200-ct657 was inserted into pBluscript KS(+) vector by using T4 DNA ligase(Promega). As a result, recombinant plasmids(referred as "pBC-Pv200-ct657" hereafter) were produced. Then, E. coli strain JM 109 was transformed with pBC-Pv200-ct657 for storage.

### Example 2-3. Preparation of expression vector pELK-MSP

Polymerase chain reaction was performed using pBC-Pv200-ct657 prepared in Example 2-2 as a template and a pair of primers, sense primer and anti-sense primer, having SEQ. ID. NO:12 and SEQ. ID. NO:13, respectively. It was confirmed that the amplified DNA fragment had a size of approximately 330 bp through electrophoresis in 1% agarose gel.

The amplified DNA fragments were purified with phenol/chloroform mixture to produce DNA fragments with high purity. The DNA fragment and vector, pET-19b (Novagen) were cleavaged with NdeI and BamHI. Then, the DNA fragment was inserted into pET-19b to prepare an expression vector, pELK-MSP having a size of approximately 6.0 kb (Refer to Figure 4).

E. coli strain BL21 (DE3)lysS was transformed with pELK-MSPs by the treatment with CaCl₂. The transformants were deposited in Korean Collection for Type Cultures of Korea Research Institute of Bioscience and Biotechnology deposited on December 18, 1999 (Deposit No. KCTC 0936BP)

### Example 2-4. mass production and purification of PV200C polypeptide in E. coli

The transformants pELK-MSP/strain BL21 (KCTC 0936BP) were cultivated for 12 hours in LB medium containing 50 µg/ml of chloramphenicol and 100 µg/ml of amphiciline. Then, 50 ml of the resulting cultures were again inoculated in 1L of LB medium and were cultivated for about 2 hours at 37°C. When absorbance(OD 600) of the cultures became 0.3, IPTG (Isopropyl thio-β-D-galactoside) was added to the medium to a final concentration of 0.2 mM. Then, the cultures were cultivated further for 7 hours.

After the cultivation, only the cells were isolated by centrifugation. The obtained cells were suspended in 30 ml of Phosphate Buffered Saline(PBS). Then, E. Coli in the suspension was crushed with Sonicator(Branson, Sonifier 450) and centrifuged at 5000 rpm. Because a part of expressed MSP proteins was in the supernatant and the remainder was in the pellet, both the supernatant and the pellet were completely suspended in 4M urea buffer(pH 7.5) where 4M urea was dissolved in PBS, and then were centrifuged, by which only the supernatant was collected.

Because the overexpressed surface protein has His marker consisting of 10 histidines in N-terminus thereof, the protein was adsorbed on histidine affinity column(Invitrogen) for purification. The contaminants which were not adsorbed on column were removed repeatedly with a washing buffer in which 10mM imidazol and 0.1M NaCl were dissolved in PBS. Then, the surface proteins adsorbed on column were desorbed and separated with an elution buffer in which 1mM imidazol and 0.1M NaCl were dissolved in PBS. As shown in Figure 5, it was confirmed by 15% SDS PAGE that the surface proteins having approximately 90% or more of purity were separated by the process.

Then, it was confirmed that PV200C polypeptide having approximately 99% or more of high purity were obtained from recombinant E.Coli by concentrating the separated solution and performing Sephacryl S-200 (Pharmacia Co., Sweden) gel filtration chromatography with the concentrate.

### Example 2-5. assay of antigenicity of the surface protein of malarial Protozoa purified from E. coli

Western Blotting(Towbin et al, Proc. Natl. Acad. Sci. 76, 43504354, 1979) was performed with the serum of the malaria patient to assay antigenicity of the MSP PV200C prepared in Example 2-4.

The PV200C prepared in Example 2-4 was loaded into 15% SDS-PAGE gel with a concentration of 5 µg per well and electrophoresed. After the electrophoresed gel was immersed in a transfer buffer solution containing 25mM Tris-HCl, 190 mM glycine (pH 8.3) and 20% methanol for 30 minutes, the protein was transferred to nitrocellulose membrane(Hafer Transphor Power Lid, Pharmacia Co.). Then, the protein-bound nitrocellulose membrane was blocked for 1 hour at ambient temperature in a blocking buffer(3% Skim milk /PBS, pH 7.4). The serum of the malaria patient diluted to 1/100 was added to the membrane and was incubated for 1 hour at ambient temperature.

Thereafter, the nitrocellulose membrane was washed 5 times at 5 minute-intervals with a washing buffer solution(0.05% Tween/PBS). The second antibody (Vector Labs, U.S.A.) to which HRP was conjugated was diluted to 1/2000, added to the membrane and incubated for 1 hour at ambient temperature. After the incubation, the nitrocellulose membrane was washed with the washing buffer solution, and then both 4-chloro-naphthol and hydrogen peroxide were added to the membrane to induce color development.

As a result, it was confirmed that only the surface protein PV200C polypeptide was blotted at 15 kDa position. Therefore, it was confirmed that the surface protein PV200C polypeptide expressed and purified by the preparation method of the present invention was an antigen having high specificity. (Refer to Figure 6)

### Example 3-1. Preparation of well plate coated with the surface antigens of malarial Protozoa

To provide a solid support to be used for the diagnosis of malaria, the solution of recombinant surface antigens of malarial Protozoa prepared in Example 1-4 or 2-4 was diluted with 0.1M carbonate buffer (pH 9.5) to 0.5 µg/ml, and 100 µl of the solution was added to each well of 96-well plate. To adsorb the surface antigens on the well, the well plate was sealed tightly and left for 18 hours at 4 °C. Then, the surface antigens which were not adsorbed on the well were removed by adding 300 µl of phosphate buffer solution containing 5% general goat serum to each well and leaving it for 18 hours at 4 °C. After the solution that remained in the well was discarded, moisture was removed by leaving the well plate for 1 hour at ambient temperature. Then, the well plate was transferred to a hermetic container with a dehumidifying agent and stored in a refrigerator at 4 °C.

In the embodiment of the present invention, the above-prepared well plate coated with the surface antigens of malarial Protozoa was used as a solid support in a diagnostic reagent and immunoassay for malaria.

### Example 3-2. Diagnosis of malaria by indirect enzyme immunoassay

To diagnose malaria by indirect enzyme immunoassay, the solid support prepared by coating 96-well plate(Nunc) with recombinant surface antigens prepared in Example 1-4 or 2-4 according to the method of Example 3-1, and the labeled antigen conjugate consisting of HRP and anti-human IgM antibody or anti-human IgG antibody derived from goat were used.

100 µl of a sample dilution solution containing 1.15 mg/ml NaOH, 0.2 mg/ml of KH₂PO₄, 0.2 mg/ml KCl, 8 mg/ml NaCl, 300 µl/ml bovine serum, 0.2 mg/ml thimerosal and the like was added to each well of 96-well plate(Nunc) coated with recombinant surface antigens according to the method of Example 3-1. Then, 10 µl of antibody-negative plasma sample or 10 µl of antibody-positive plasma sample was added to the wells, and the resulting mixture were well mixed. The well plate was incubated for 60 minutes in a reactor at 37 °C. After the reaction terminated, the well plate was washed 5 times with 300 µl of a phosphate buffer solution containing 0.05% Tween 20. Anti-human IgG antibody(Cappel) or anti-human IgM antibody(Bethyl) derived from goat to which HRP was conjugated was diluted to 1/40,000 with a dilution solution containing 10mM Tris, 0.5M NaCl, 1mM CaCl₂, 0.5% glycerol, 0.2 ml/ml bovine serum, 100 µl/ml goat serum, 0.05% Tween 20, 0.2 mg/ml thimerosal and 50 µg/ml phenol red. 100 µl of the diluted conjugate was added to each well of the 96-well plate and the well plate was incubated for 60 minutes at 37 °C. After the reaction terminated, the well plate was washed 5 times with 0.05% Tween 20/phosphate buffer solution. 100 µl of a substrate solution containing 100 µg/ml tetramethylbenzidine, 0.006% hydrogen peroxide and citrate-phosphate buffer solution(pH 4.5) was added to each well. After color development in a dark place for 30 minutes, 100 µl of the reaction-stopping solution (2N sulfuric acid solution) was added to each well to terminate the color development reaction. Then, the absorbance was measured at 450 nm (reference wavelength, 650 nm) by using the 96 well plate reader (Molecular Devices, USA). Tetramethylbenzidine that is a color fixing agent in the substrate solution was decomposed by HRP conjugated to anti-human IgG antibody or anti-human IgM antibody, to induce the color development. Then, existence of malaria-specific antibodies and amount of the antibodies were detected by measuring the absorbance.

According to the above method, 20 malaria-positive samples(plasma of the malaria patients) and 48 malaria-negative samples(plasma of normal people) were diagnosed for malaria. When positive cut-off value was determined by adding 0.5 to the mean of the negative samples, according to the indirect enzyme immunoassay using the recombinant surface antigens of malarial Protozoa according to the present invention, 18 out of the 20 malaria patients' samples were judged to be positive and 46 out of 48 normal people's samples were judged to be negative. Therefore, the indirect enzyme immunoassay of the present invention exhibited 90% of sensitivity and 95.8% of specificity.

### Example 3-3. Preparation of the surface antigen of malarial Protozoa conjugated to HRP

The labeled antigen conjugates to be used for antigen sandwich enzyme immunoassay were prepared.

The recombinant surface antigens of malarial Protozoa prepared in Example 1-4 or 2-4 was dialyzed for 1 day at 4 °C against 1 L of 0.01 M sodium carbonate buffer solution(pH 9.6). The buffer solution was exchanged 3 times during the dialysis.

Also, 5 mg of HRP was dissolved in 0.5 ml of distilled water in a tube, and then 100 µl of 42 mg/ml NaIO₄ was added to the HRP solution. After the tube was wrapped with foil, it was shaken for 30 minutes at ambient temperature for the oxidation reaction. After HRP was oxidized enough, 60 µl of 1M glycerol was added to the reaction solution. Thereafter, the tube wrapped with foil was shaken for 30 minutes at ambient temperature to terminate the oxidation reaction.

For a conjugation reaction of HRP and the surface antigen, salts were removed from the HRP reaction solution by PD 10 column (Pharmacia) saturated with 0.01 M sodium carbonate buffer (pH 9.6). The above solution of the surface antigens was added to the oxidized HRP reaction solution. Then, the tube was wrapped with foil and shaken overnight at ambient temperature to prepare conjugates of HRPs and the surface antigens. After the conjugation reaction of HRP and the surface antigen was terminated, in order to stabilize the HRP-surface antigen conjugates, 40.8 µl of 4 mg/ml NaBH₄ solution was added to the tube, and the tube wrapped with foil was shaken at 4 °C for 2 hours for the reaction.

To remove HRPs in the reaction solution which were not conjugated, the surface proteins conjugated to HRPs were adsorbed on Probond column that was equilibrated with phosphate buffer solution by utilizing histidine residues linked with C-terminus of the surface protein. Then, HRPs which were not conjugated were removed by phosphate buffer solution. The HRP-surface antigen conjugates adsorbed on Probond column were desorbed by phosphate buffer solution containing 1M imidazole. The conjugates were collected. The protein concentration of the collected HRP-surface antigen conjugates was determined by using commercially available BSA protein quantitative test kit. The sample was stored in a refrigerator after addition of bovine serum albumin (BSA) of final concentration of 1 % .

The above-prepared HRP-surface antigen conjugates were used as labeled antigen conjugates in an antigen sandwich enzyme immunoassay and diagnostic reagent for malaria.

### Example 3-4. Diagnosis of malaria by antigen sandwich enzyme immunoassay using HRP-surface antigen conjugates

Antigen sandwich enzyme immunoassay for malaria was performed by using the HRP-surface antigen conjugates prepared in Example 3-3.

30 µl of the solution of the HRP-surface antigen conjugates was added to each well of 96-well plate(Nunc) coated with recombinant surface antigens according to the method of Example 3-1. Then, 100 µl of malaria-positive samples(plasmas of malaria patients) or 100 µl of malaria-negative samples(plasmas of normal people) was added to the wells, and the resulting mixtures were well mixed. The well plate was incubated for 90 minutes in a reactor at 37°C. After the reaction terminated, the well plate was washed 5 times with each 300 µl of a phosphate buffer solution containing Tween 20. 100 µl of a substrate solution containing 100 µg/ml tetramethylbenzidine, 0.006% hydrogen peroxide and citrate-phosphate buffer solution(pH 4.5) was added to each well of the well plate. After color development of the plate in a dark place for 30 minutes, 100 µl of the reaction-stopping solution (2N sulfuric acid solution) was added to each well to terminate the color development reaction. Then, the absorbance was measured at 450 nm (reference wavelength, 650 nm) by using the 96 well plate reader (Molecular Devices, USA). Tetramethylbenzidine that is a color fixing agent in the substrate solution was decomposed by HRP conjugated to the surface antigen, to induce the color development. Then, existence of malaria-specific antibodies and amount of the antibodies were detected by measuring the absorbance.

According to the above method, 202 malaria-positive samples(plasmas of malaria patients) and 400 malaria-negative samples(plasmas of normal people) were diagnosed for malaria. When positive cut-off value was determined by adding 0.5 to the mean of the negative samples, according to the antigen sandwich enzyme immunoassay using the HRP-surface antigen conjugates according to the present invention, 199 out of 202 malaria patients' samples were judged to be positive and 398 out of the 400 normal people's samples were judged to be negative. Therefore, the antigen sandwich enzyme immunoassay of the present invention exhibited 98.5% of sensitivity and 99.6% of specificity.

### Example 4-1. Diagnosis of malaria by IgM capture enzyme immunoassay

To diagnose malaria by IgM capture enzyme immunoassay, a solid support coated with anti-human IgM antibodies derived from goat, and the labeled antigen conjugates consisting of HRP and recombinant surface antigens purified from yeast or E.Coli transformant were used.

The plate was prepared as follows: anti-human IgM antibodies (anti-human IgM mu chain-specific, affinity purified, Bethyl Co., USA) derived from goat were diluted with phosphate buffer solution (pH 7.0) to 1~10 µg/ml and then 100~200 µl of the mixture was added to each well of the polystyrene plate. The well plate was left for approximately 12~18 hours at ambient temperature, and the solution that remained in the well was removed by suction. 250 µl of 0.1 % gelatin or 0.1 % casein dissolved in phosphate buffer solution was added to each well, and the well plate was left for 2 hours at ambient temperature. Then, the solution that remained in the well was removed by suction. The well plate was dried for 18 hours in a cold room and used.

100 µl of a sample dilution solution that is a phosphate buffer solution containing 0.1 % bovine serum albumin was added to each well of the well plate coated with anti-human IgM antibodies derived from goat. Then, 2~20 µl of antibody-negative plasma sample or 2~20 µl of antibody-positive plasma sample was added to the wells, and the resulting mixtures were well mixed. The well plate was incubated for 60 minutes in a reactor at 37 °C. After the reaction terminated, the well plate was washed 5 times with 300 µl of a phosphate buffer solution containing 0.05% Tween 20.

The surface antigens of malarial Protozoa purified from yeast or E.Coli transformant which were prepared in Example 1-4 or 2-4 were conjugated with HRPs according to the method of Example 3-3 and then used.

The above-prepared conjugates consisting of HRPs and the surface antigens were diluted to 1:100 to 1:1,000 with phosphate buffer solution (pH 7.0) containing 1% BSA and 0.05% Tween 20. Then, 100 µl of the solution of the HRP-surface antigen conjugates was added to each well of the well plate. The well plate was incubated for 30 minutes at 37 °C. After the reaction terminated, the well plate was washed 5 times with phosphate buffer solution containing 0.05% Tween 20. 100 µl of a substrate solution containing 100 µg/ml tetramethylbenzidine, 0.006% hydrogen peroxide and citrate-phosphate buffer solution(pH 4.5) was added to each well of the plate. After color development in a dark place for 30 minutes, 100 µl of the reaction-stopping solution (1N sulfuric acid solution) was added to each well to terminate the color development reaction. Then, the absorbance was measured at 450 nm (reference wavelength, 650 nm) by using the 96 well plate reader (Molecular Devices, USA). Tetramethylbenzidine that is a color fixing agent in the substrate solution was decomposed by HRP of the labeled antigen conjugate, to induce the color development. Then, existence of malaria-specific IgM and amount of the IgM were detected by measuring the absorbance.

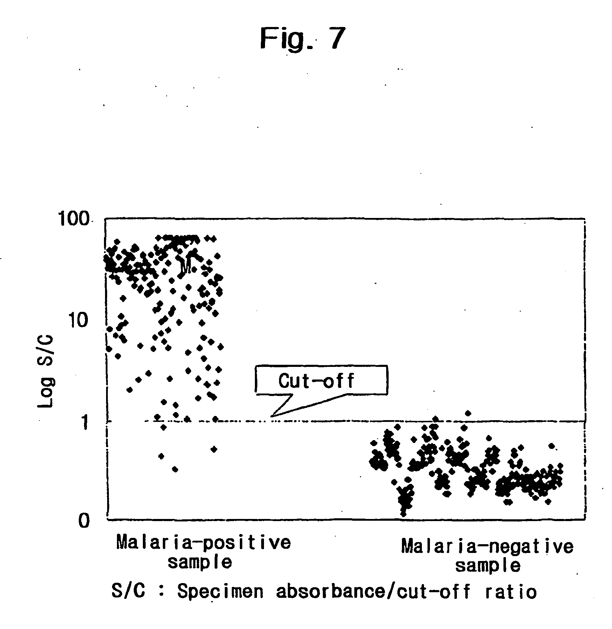

According to the above method, 216 malaria-positive samples(plasmas of malaria patients) and 353 malaria-negative samples(plasmas of normal people) were diagnosed for malaria. When positive cut-off value was determined by adding 0.05 to the mean of the negative samples, according to IgM capture enzyme immunoassay using the recombinant surface antigen of malarial Protozoa according to the present invention, 212 out of 216 malaria patients' samples were judged to be positive and 351 of 353 normal people's samples were judged to be negative. Therefore, the IgM capture enzyme immunoassay of the present invention exhibited 98.1 % of sensitivity and 99.4% of specificity (Refer to Figure 7).

### Example 4-2. Comparison of IgM capture enzyme immunoassay with indirect enzyme immunoassay

In the IgM capture enzyme immunoassay, a solid support coated with anti-human IgM antibodies derived from goat, and the labeled antigen conjugates consisting of HRPs and the recombinant surface antigens purified from yeast or E.Coli transformant were used. The IgM capture enzyme immunoassay was performed according to the method of Example 4-1.

Also, the indirect enzyme immunoassay was performed according to the method of Example 3-2.

To compare the IgM capture enzyme immunoassay with the indirect enzyme immunoassay, 75 malaria-positive samples(plasmas of malaria patients) and 92 malaria-negative samples(plasmas of normal people) were diagnosed for malaria. When positive cut-off value was determined by adding 0.05 to the mean of the negative samples, according to the IgM capture enzyme immunoassay using the recombinant surface antigens of malarial Protozoa according to the present invention, 73 out of 75 malaria patients' samples were judged to be positive and 92 of 92 normal people's samples were judged to be negative. Therefore, the IgM capture enzyme immunoassay of the present invention exhibited 97.3% of sensitivity and 100% of specificity.

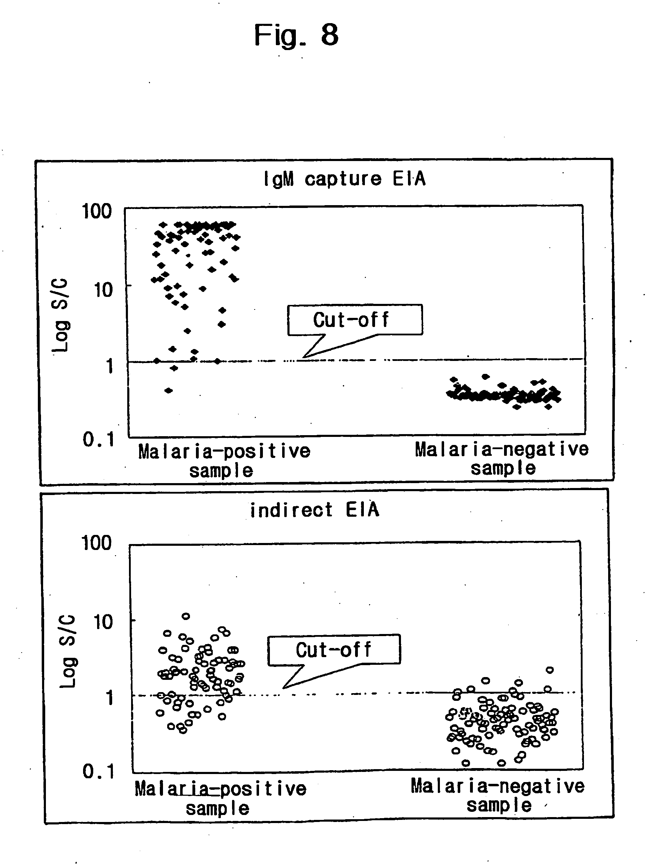

In the meantime, when positive cut-off value was determined by adding 0.2 to the mean of the negative samples, according to the indirect enzyme immunoassay of the present invention, 59 out of 75 malaria patients' samples were judged to be positive and 85 of 92 normal people's samples were judged to be negative. Therefore, the indirect enzyme immunoassay of the present invention exhibited 78.6% of sensitivity and 92.4% of specificity. (Refer to Figure 8).

### Example 4-3. Comparison of IgM capture enzyme immunoassay with antigen sandwich enzyme immunoassay in normal people classified by age

In the IgM capture enzyme immunoassay, a solid support coated with anti-human IgM antibodies derived from goat, and the labeled antigen conjugates consisting of HRPs and the recombinant surface antigens purified from yeast or E.Coli transformant were used. The IgM capture enzyme immunoassay was performed according to the method of Example 4-1.

Also, the antigen sandwich enzyme immunoassay was performed according to the method of Example 3-4.

To compare the IgM capture enzyme immunoassay with the antigen sandwich enzyme immunoassay, plasma samples of 129 normal people were diagnosed for malaria. The 129 normal people consisted of 26 people in teen, 43 people in twenties, 33 in thirties and 27 in forties.

When positive cut-off value was determined by adding 0.05 to the mean of the negative samples, according to the IgM capture enzyme immunoassay using the recombinant surface antigens of malarial Protozoa according to the present invention, 129 of 129 normal people's samples were judged to be negative. Therefore, the IgM capture enzyme immunoassay of the present invention exhibited 100% of specificity.

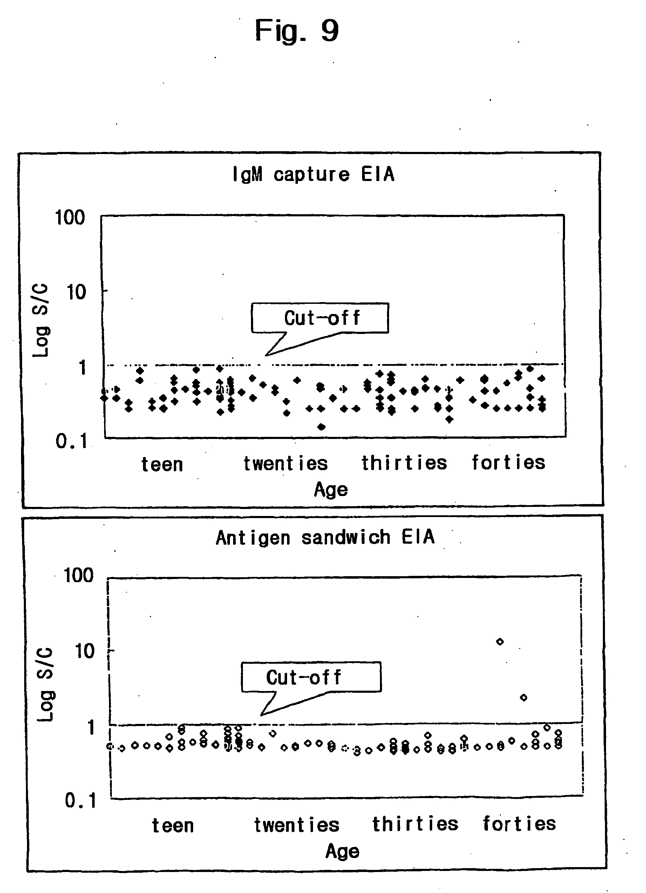

In the meantime, when positive cut-off value was determined by adding 0.05 to the mean of the negative samples, according to the antigen sandwich enzyme immunoassay of the present invention, 127 of 129 normal people's samples were judged to be negative. Therefore, the antigen sandwich enzyme immunoassay of the present invention exhibited 98.4% of specificity. Especially, when plasma samples of normal people in thirties or less who were born after the prevalence period of malaria were diagnosed for malaria, the antigen sandwich enzyme immunoassay of the present invention exhibited 100% of specificity. However, when plasma samples of normal people in forties who were born in the prevalence period of malaria were diagnosed for malaria, 2 of 27 normal people's samples were judged to be positive and the antigen sandwich enzyme immunoassay of the present invention exhibited 92.6% of specificity. Therefore, the specificity of the antigen sandwich enzyme immunoassay of the present invention decreased in specific ages (Refer to Figure 9).

## Claims

1. A diagnostic method for detecting malaria-specific antibodies in blood, comprising the steps of:
(a) immobilizing a surface antigen of a part or whole of PV200C polypeptide in the C-terminus of Merozoite Surface Protein of Plasmodium Vivax on a solid support;
(b) pouring the blood or plasma sample;
(c) adding labelled antigen conjugates consisting of a part or whole of PV200C polypeptide in the C-terminus of Merozoite Surface Protein of Plasmodium Vivax; and
(d) inducing colour development by using a substrate solution containing a colour fixing agent, wherein said PV200C polypeptide is the amino acid sequence as shown in SEQ ID No: 1.

2. A diagnostic method for detecting malaria-specific IgM in blood, comprising the steps of:
(a) immobilising anti-human IgM antibodies on a solid support
(b) pouring blood serum or plasma sample;
(c) adding labelled antigen conjugates consisting of markers and antigens of a part or whole of PV200C polypeptide in the C-terminus of Merozoite Surface Protein of Plasmodium Vivax and marker; and
(d) inducing colour development by using a substrate solution containing a colour fixing agent, wherein said PV200C polypeptide is the amino acid sequence as shown in SEQ ID No: 1.

3. A diagnostic reagent for malaria detecting malaria-specific antibodies in blood, comprising:
a solid support coated with surface antigens of a part or whole of PV200C polypeptide in the C-terminus of Merozoite Surface Protein of Plasmodium Vivax;
labeled antigen conjugated consisting of a part or whole of PV200C polypeptide in the C-terminus of Merozoite Surface Protein of Plasmodium Vivax;
and markers; and
a substrate solution containing a color fixing agent, wherein said PV200C polypeptide is the amino acid sequence as shown in SEQ ID No: 1.

4. A diagnostic reagent for malaria detecting malaria-specific antibodies in blood, comprising:
a solid support coated with anti-human IgM antibodies;
labeled antigen conjugate consisting of markers and antigens of part or whole of PV200C polypeptide in the C-terminus of Merozoite Surface Protein of Plasmodium Vivax;
and
a substrate solution containing a color fixing agent, wherein said PV200C polypeptide is the amino acid sequence as shown in SEQ ID No: 1.

## Patentansprüche

1. Diagnoseverfahren zum Nachweis Malaria-spezifischer Antikörper im Blut, das folgende Schritte umfasst:
(a) Immobilisierung eines Oberflächenantigens des ganzen PV200C-Polypeptids im C-Terminus des Merozoiten-Oberflächenproteins von Plasmodium vivax oder eines Teils davon auf einem festen Träger;
(b) Einfüllen einer Blut- oder Plasmaprobe;
(c) Zugabe markierter Antigenkonjugate, bestehend aus dem ganzen PV200C-Polypeptid im C-Terminus des Merozoiten-Oberflächenproteins von Plasmodium vivax oder einem Teil davon und Marker; und
(d) Induktion einer Farbentwicklung durch den Einsatz einer Substratlösung, die einen Farbfixierer enthält, wobei das PV200C-Polypeptid die in SEQ ID NO: 1 gezeigte Aminosäuresequenz besitzt.

2. Diagnoseverfahren zum Nachweis Malaria-spezifischer IgM im Blut, das folgende Schritte umfasst:
(a) Immobilisierung anti-humaner IgM-Antikörper auf einem festen Träger;
(b) Einfüllen einer Blutserum- oder Plasmaprobe;
(c) Zugabe markierter Antigenkonjugate, bestehend aus Markern und Antigenen des ganzen PV200C-Polypeptids im C-Terminus des Merozoiten-Oberflächenproteins von Plasmodium vivax oder eines Teils davon; und
(d) Induktion einer Farbentwicklung durch den Einsatz einer Substratlösung, die einen Farbfixierer enthält, wobei das PV200C-Polypeptid die in SEQ ID NO: 1 gezeigte Aminosäuresequenz besitzt.

3. Diagnostisches Reagens zum Nachweis Malaria-spezifischer Antikörper im Blut, umfassend:
einen festen Träger, beschichtet mit Oberflächen-Antigenen des ganzen PV200C-Polypeptids im C-Terminus des Merozoiten-Oberflächenproteins von Plasmodium vivax oder eines Teils davon;
markierte Antigenkonjugate, bestehend aus dem ganzen PV200C-Polypeptid im C-Terminus des Merozoiten-Oberflächenproteins von Plasmodium vivax oder einem Teil davon; und Markern; und
ein Substratlösung, die einen Farbfixierer enthält, wobei das PV200C-Polypeptid die in SEQ ID NO: 1 gezeigte Aminosäuresequenz besitzt.

4. Diagnostisches Reagens zum Nachweis Malaria-spezifischer Antikörper im Blut, umfassend:
einen festen Träger, beschichtet mit anti-humanen IgM-Antikörpern; markierte Antigenkonjugate, bestehend aus Markern und Antigenen des ganzen PV200C-Polypeptids im C-Terminus des Merozoiten-Oberflächenproteins von Plasmodium vivax oder eines Teils davon; und
eine Substratlösung, die einen Farbfixierer enthält, wobei das PV200C-Polypeptid die in SEQ ID NO: 1 gezeigte Aminosäuresequenz besitzt.

## Revendications

1. Procédé de diagnostic permettant de détecter des anticorps spécifiques du paludisme dans le sang, comprenant les étapes suivantes :
(a) immobilisation d'un antigène de surface d'une partie ou de la totalité du polypeptide PV200C dans la terminaison C de la protéine de surface d'un mérozoïte de *Plasmodium Vivax* sur un support solide ;
(b) versement de l'échantillon de sang ou de plasma ;
(c) ajout des conjugués d'antigène marqués constitués d'une partie ou de la totalité du polypeptide PV200C dans la terminaison C de la protéine de surface d'un mérozoïte de *Plasmodium Vivax* et d'un marqueur ; et
(d) induction d'un développement de couleur en utilisant une solution de substrat contenant un agent fixant la couleur, dans lequel ledit polypeptide PV200C est la séquence d'acides aminés présentée dans SEQ ID N° : 1.

2. Procédé de diagnostic permettant de détecter une IgM spécifique du paludisme dans le sang, comprenant les étapes suivantes :
(a) immobilisation d'anticorps d'IgM anti-humain sur un support solide ;
(b) versement de l'échantillon de sérum sanguin ou de plasma ;
(c) ajout des conjugués d'antigène marqués constitués de marqueurs et d'antigènes d'une partie ou de la totalité du polypeptide PV200C dans la terminaison C de la protéine de surface d'un mérozoïte de *Plasmodium Vivax* ; et
(d) induction d'un développement de couleur en utilisant une solution de substrat contenant un agent fixant la couleur, dans lequel ledit polypeptide PV200C est la séquence d'acides aminés présentée dans SEQ ID N° : 1.

3. Réactif de diagnostic du paludisme permettant de détecter des anticorps spécifiques du paludisme dans le sang, comprenant :
un support solide revêtu d'antigènes de surface d'une partie ou de la totalité du polypeptide PV200C dans la terminaison C de la protéine de surface d'un mérozoïte de *Plasmodium Vivax* ;
l'antigène marqué conjugué constitué d'une partie ou de la totalité du polypeptide PV200C dans la terminaison C de la protéine de surface de mérozoïte de *Plasmodium Vivax* ;
et des marqueurs ; et
une solution de substrat contenant un agent fixant la couleur, ledit polypeptide PV200C étant la séquence d'acides aminés présentée dans SEQ ID N° : 1.

4. Réactif de diagnostic du paludisme permettant de détecter des anticorps spécifiques du paludisme dans le sang, comprenant :
un support solide revêtu d'anticorps d'IgM anti-humains ;
le conjugué d'antigène marqué constitué de marqueurs et d'antigènes d'une partie ou de la totalité du polypeptide PV200C dans la terminaison C de la protéine de surface d'un mérozoïte de *Plasmodium Vivax*;
et
une solution de substrat contenant un agent fixant la couleur, ledit polypeptide PV200C étant la séquence d'acides aminés présentée dans SEQ ID N° : 1.
